# EUROPEAN PATENT SPECIFICATION

(11) **EP 1 704 167 B1**
(45) Date of publication and mention of the grant of the patent: **12.01.2011**
(21) Application number: 04820430.9
(22) Date of filing: 10.12.2004
(51) Int. Cl.: C07K 19/00, C07K 14/16, C07K 14/11, A61K 39/12, G01N 33/68

(54) **A METHOD TO MAKE A PEPTIDE-CARRIER CONJUGATE WITH A HIGH IMMUNOGENICITY**
VERFAHREN ZUR HERSTELLUNG EINES PEPTID-TRÄGER-KONJUGATS MIT HOHER IMMUNOGENIZITÄT
PROCEDE DE PRODUCTION D'UN CONJUGUE PORTEUR DE PEPTIDES A ANTIGENICITE ELEVEE

(30) Priority: 18.12.2003 US 530867 P
(43) Date of publication of application: 27.09.2006
(73) Proprietor: ISTITUTO DI RICERCHE DI BIOLOGIA MOLECOLARE P. ANGELETTI S.P.A., 00040 Pomezia (IT)
(72) Inventor: BIANCHI, Elisabetta, 00040 Pomezia (Rome) (IT); FINOTTO, Marco, 00040 Pomezia (Rome) (IT); INGALLINELLA, Paolo, 00040 Pomezia (Rome) (IT); PESSI, Antonello, 00040 Pomezia (Rome) (IT)
(74) Representative: Buchan, Gavin MacNicol
(86) International application number: PCT/EP2004/014160
(87) International publication number: WO 2005/058968

(56) References cited:
- EP-A1- 0 519 554
- EP-A1- 0 519 554
- WO-A-01/93804
- GB-A- 2 271 995
- US-A- 5 606 030
- US-A- 5 606 030
- US-A- 5 763 574
- ZANGWILL K M ET AL: "Safety and immunogenicity of a heptavalent pneumococcal conjugate vaccine in infants" VACCINE, BUTTERWORTH SCIENTIFIC. GUILDFORD, GB, vol. 21, no. 17-18, 16 May 2003 (2003-05-16), pages 1894-1900, XP004421101 ISSN: 0264-410X

### SEQUENCE LISTING

<110> Merck & Co., Inc.
<120> A METHOD TO MAKE A PEPTIDE-CARRIER CONJUGATE WITH A HIGH IMMUNOGENICITY
<130> PCT ITR0054.
<150> 60/530,867
   <151> 2003-12-18
<160> 11
<170> FastSEQ for Windows Version 4.0
<210> 1
   <211> 23
   <212> PRT
   <213> Artificial Sequence
<220>
   <223> Artificial Sequence
<400> 1
   Cys Gly Pro Glu Lys Gln Thr Arg Gly Leu Phe Gly Ala Ile Ala Gly
   1 5 10 15
   Phe Ile Glu Asn Gly Asn His
   20
<210> 2
   <211> 24
   <212> PRT
   <213> Artificial Sequence
<220>
   <223> Artificial seq.
<400> 2
   Ala Cys Gly Pro Glu Lys Gln Thr Arg Gly Leu Phe Gly Ala Ile Ala
   1 5 10 15
   Gly Phe Ile Glu Asn Gly Glu His
   20
<210> 3
   <211> 22
   <212> PRT
   <213> Artificial Sequence
<220>
   <223> Artificial seq.
<400> 3
   Pro Glu Lys Gln Thr Arg Gly Leu Phe Gly Ala Ile Ala Gly Phe Ile
   1 5 10 15
   Glu Asn Gly Cys Asn His
   20
<210> 4
   <211> 23
   <212> PRT
   <213> Artificial Sequence
<220>
   <223> Artificial seq.
<400> 4
   Ser Glu Pro Glu Lys Gln Thr Arg Gly Leu Phe Gly Ala Ile Ala Gly
   1 5 10 15
   Phe Ile Glu Asn Gly Cys His
   20
<210> 5
   <211> 20
   <212> PRT
   <213> Artificial Sequence
<220>
   <223> Artificial seq.
<400> 5
   Ser Thr Met Gly Ala Arg Ser Met Thr Leu Thr Val Gln Ala Arg Gln
    1               5                  10                  15
   Leu Cys Asn His
               20
<210> 6
   <211> 21
   <212> PRT
   <213> Artificial Sequence
<220>
   <223> Artificial seq.
<400> 6
   Ser Ser Thr Met Gly Ala Arg Ser Met Thr Leu Thr Val Gln Ala Arg
    1               5                  10                  15
   Gln Leu Cys Asn His
               20
<210> 7
   <211> 29
   <212> PRT
   <213> Artificial Sequence
<220>
   <223> Artificial seq.
<400> 7
   Gly Leu Phe Gly Ala Ile Ala Gly Phe Ile Glu Asn Gly Trp Glu Gly
    1               5                  10                  15
   Met Ile Asp Gly Gly Cys Gly Lys Lys Lys Lys Asn His
               20                  25
<210> 8
   <211> 23
   <212> PRT
   <213> Artificial Sequence
<220>
   <223> Artificial seq.
<400> 8
   Gly Leu Phe Gly Ala Ile Ala Gly Phe Ile Glu Asn Gly Trp Glu Gly
   1 5 10 15
   Met Val Asp Gly Cys Glu His
   20
<210> 9
   <211> 16
   <212> PRT
   <213> Artificial Sequence
<220>
   <223> Artificial seq.
<400> 9
   Gly Leu Phe Gly Ala Ile Ala Gly Phe Ile Glu Asn Gly Cys Glu His
   1 5 10 15
<210> 10
   <211> 17
   <212> PRT
   <213> Artificial Sequence
<220>
   <223> Artificial seq.
<400> 10
   Ala Gly Leu Phe Gly Ala Ile Ala Gly Phe Ile Glu Asn Gly Cys Glu
   1 5 10 15
   His
<210> 11
   <211> 17
   <212> PRT
   <213> Artificial Sequence
<220>
   <223> Artificial seq.
<400> 11
   Ser Gly Leu Phe Gly Ala Ile Ala Gly Phe Ile Glu Asn Gly Cys Glu
   1 5 10 15
   His

## Description

### FIELD OF THE INVENTION

The present invention relates to a method of increasing the immunogenicity of a peptide. The present invention also relates to a method of increasing both the peptide load and the solubility of the conjugate of the peptide and a carrier.

### BACKGROUND OF THE INVENTION

Peptides are widely used in vaccination and production of antibodies from animals. Because of their low molecular weight, peptides are generally not sufficient to elicit immune response by themselves. To become effective immunogens, peptides generally need to be conjugated to a carrier protein, such as OMPC (outer membrane protein complex of *Neisseria meningitidis*), through a covalent linkage.

One crucial characteristic of the conjugate is the peptide load, defined as the number of moles of peptide per mole of the carrier. Currently, there are two main obstacles for obtaining a high peptide load: (i) solubility of the ensuing conjugate, and (ii) solubility of the peptide. These properties are not independent, and manipulations, which improve the latter, can be detrimental to the former. Hence, it is often difficult to obtain a high peptide load.

For instance, a poor solubility of OMPC conjugates was observed when-conjugating OMPC and HIV peptide sequences containing one or more basic amino acids (arginine, histidine and lysine), using thiolactones. To improve the low solubility, Leanza et al. reported the use of acidic derivatives of thiolactones, as a means of neutralizing excess positive charges introduced by the peptide epitopes. However the maximum thiolation levels on OMPC, using anionic thiolactones, is only about 60% of that achieved by a non-charged thiolactone. There seems to be a limit in the amount of acidic thiolactone moieties that can be incorporated, and it is possible to fully achieve thiolation of OMPC only by using a mixture of acidic and non-charged thiolactones. This results in OMPC being derivatized with two different linkers, one of which renders it still susceptible to precipitation upon peptide conjugation. To avoid conjugate precipitation, therefore, the optimal conditions for peptide loading have to be set each time. (Leanza et al. Acidic Derivatives of Homocysteine Thiolactone: Utility as Anionic Linkers. Bioconjugate Chem. 3(1992) 514-518; U.S. Pat. No. 5,274,122)

Another method to overcome precipitation of peptide-OMPC conjugates devised a co-conjugation strategy, wherein about a half of the thiol groups of thiolated OMPC were first captured with maleimido-propionic acid (with a resident anion) and the balance of the thiol groups were allowed to react with (cationic) Arg-containing-HIV peptides. The resulting charge-balanced conjugates had suitable aqueous solubility and could be formulated for use as vaccines. This method has the disadvantage of reducing the potential peptide loads by 50%, and has not been shown to be a general way of overcoming the problem for any peptide sequence (Tolman et al. Int. J. Peptide Protein Res. 41 (1993) 455-466; U.S. Pat. No. 5,606,030).

Hence, there is a need to develop a method of increasing both the peptide load of a peptide-carrier conjugate and the solubility of the conjugate. A procedure that would enable the synthesis of peptide-carrier conjugates high peptide load, *irrespective* of the peptide sequence, is a major need for the development of peptide-carrier based vaccines.

### SUMMARY OF THE INVENTION

The present invention provides a method for making a peptide-carrier conjugate as described in claim 1. The method comprises modifying a first peptide to produce a second peptide so that the pI of the second peptide is lower than the pI of the first peptide, and conjugating a plurality of the second peptide to OMPC to obtain a peptide-carrier conjugate. The peptide load, or the solubility of the conjugate, or both of them are increased by the modification. The second peptide has a non-naturally occurring sequence.

According to an embodiment of the present invention, the first peptide has an amino acid sequence selected from a pathogen. According to a preferred embodiment of the present invention, the pathogen is selected from the group consisting of *Haemophilus influenza*, hepatitis viruses A, B, or C, HIV, human papilloma virus, measles, mumps, rubella, varicella, rotavirus, *Streptococcus pneumonia* and *Staphylococcus aureus.* According to a further preferred embodiment of the present invention, the first peptide has a sequence selected from sequence of HA protein of Influenza virus A or B. According to an alternative embodiment of the present invention, the first peptide has a sequence selected from HIV gp41 protein.

According to a preferred embodiment of the present invention, the first peptide is modified at or near either terminus of the first peptide sequence.

According to an embodiment of the present invention, the first peptide is modified with addition of at least one amino acid with anionic side chain to the first peptide sequence. According to a preferred embodiment of the present invention, the amino acid with anionic side chain is either glutamate or aspartate.

According to an embodiment of the present invention, the first peptide is modified at the side chain of an amino acid residue in the first peptide sequence. According to a preferred embodiment of the present invention, the modification at the side chain is selected from the group consisting of phosphorylation or sulphation of serine, threonine, tyrosine, aspartate, or histidine, carboxylation of glutamate, acylation of lysine, or oxidation of cysteine.

According to an embodiment of the present invention, the first peptide is modified at N-terminal or C-terminal of the first peptide sequence. According to a preferred embodiment of the present invention, the first peptide is an amidated peptide and is modified with carboxylation of the C terminal group. According to another preferred embodiment of the present invention, the first peptide is modified with acylation such as acetylation or succinylation of N-terminal amino group.

According to an embodiment of the present invention, the second peptide is conjugated to the carrier through cross-link between the lysine residue of the carrier and cysteine residue of the second peptide. According to a preferred embodiment of the present invention, the cross-link is achieved using sulfosuccinimidyl 4-(N-maleimidomethyl)cyclohexane-1-carboxylate (sSMCC). According to an alternative embodiment of the present invention, the cross-link is achieved using m-maleimidobenzoyl-N-dydroxysuccinimide ester (MBS).

According to another embodiment of the present invention, the second peptide is conjugated to the carrier through cross-link between the lysine residue of the carrier and a bromoacetyl residue or a maleimidyl residue of the second peptide. According to a preferred embodiment of the present invention, the cross-link is achieved using N-acetyl homocysteine thiolactone (NAHT).

According to an embodiment of the present invention, the first peptide has a molecular weight at the range from 500 Da to 30000 Da. According to a preferred embodiment of the present invention, the first peptide has a molecular weight at the range from 1400 Da to 7900 Da. According to a further preferred embodiment of the present invention, the first peptide has a molecular weight at the range from 1800 Da to 5000 Da. According to a more preferred embodiment of the present invention, the first peptide has a molecular weight at the range from 2000 Da to 4000 Da.

According to a preferred embodiment of the present invention, the peptide load of the conjugate is increased to the range from 1000 to 6000 moles/mole, more preferably from 2000 to 4000 moles/mole.

According to an embodiment of the present invention, the pI of the second peptide is lower than 6. According to a preferred embodiment of the present invention, the pI of the second peptide is in the range from 2 to 6. According to a preferred embodiment of the present invention, the pI of the second peptide is in the range from 3.5 to 5.

The present invention provides a method for determining the favorable pI range for conjugation. The method comprises conjugating a plurality of each of various peptides with a wide range of pI to an OMPC carrier, measuring the peptide load for each of the peptides, and determining the favorable pI range for the conjugation to achieve a high peptide load.

According to a preferred embodiment of the present invention, the modified peptide is selected from the group consisting of SEQ ID NO: 2, 4, 6, 8, 9, 10 and 11.

According to an embodiment of the present invention, the conjugate has a peptide load of from 2000 to 4000 moles/mole.

### BRIEF DESCRIPTION OF THE DRAWINGS

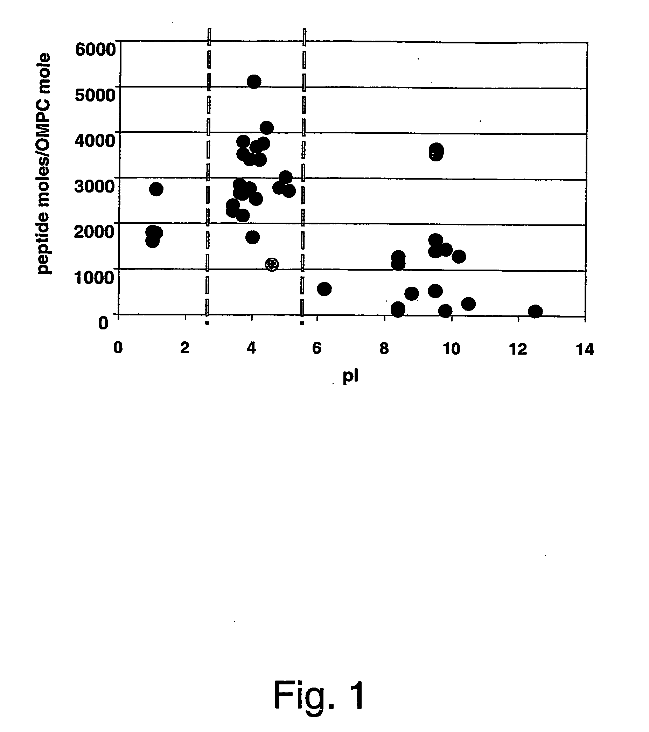

Fig. 1: Peptide load on OMPC as a function of pI of the peptide sequence.

### DETAILED DESCRIPTION OF THE INVENTION

### 1. The Adjustment of the pI of a Peptide Improves the Immunogenicity of the Conjugate of the Peptide to a Carrier

The present invention relates to methods of increasing both the peptide load and the solubility of peptide-carrier conjugates.

The present invention also provides a method for increasing the immunogenicity of peptides. The method comprises adjusting the pI of a peptide by modifying the peptide, and conjugating a plurality of the modified peptide to a carrier. As used herein, "adjusting the pI of a peptide" means changing the pI of the peptide to such a range that the peptide load and / or the solubility of the conjugate are increased.

From experiments using antigens from HIV and Influenza virus, it appears that an important requisite for a peptide-carrier conjugate to raise good antibody titers, is that the peptide load on the carrier OMPC be not less than 500-1000 moles of peptide per mole of OMPC. While with a load in excess of 500 copies, anti-peptide titers of 500.000 or higher have been observed, good antibody titers have not been observed below this threshold load. In other words, a high peptide load of the conjugate is usually essential to obtain high immunogenicity of the peptide.

In preparation of a peptide-carrier conjugate, however, the covalent addition of a peptide to a carrier such as OMPC frequently results in irreversible precipitation of the conjugate. Therefore for each peptide epitope, the maximum number of peptide moles, that can be incorporated on a mole of the carrier, i.e., the peptide load, have to be carefully controlled, in order to avoid precipitation of the ensuing conjugate. For many peptide sequences, it is not possible to achieve a good load of peptide onto the carrier.

For example, in the course of the preparation of OMPC conjugates with 29 different peptide sequences from the HIV gp41 protein and from the Influenza virus A HA protein, we found that only 55 % of the peptides could be loaded onto the carrier with peptide load well over 500 copies.

Analysis of the chemical-physical properties of the conjugated peptides did not reveal a correlation with the hydrophobicity/hydrophilicity, and with the solubility of the peptide (although of course a minimum of solubility is required to carry out the conjugation).

Our experiments with model peptides (Fig. 1), and prior observations (data not shown) suggested that peptides particularly rich in positive charges do not load well on OMPC. Many sequences that are not particularly rich in positive charges, however, can cause precipitation of the conjugate. As disclosed above, only 55% of all the tested peptides could be loaded onto OMPC in excess of 500 copies per OMPC molecule. Analysis of the peptide sequences led us to formulate the hypothesis that the negative effect on the precipitation of the OMPC conjugate is more generally related to the net charge of the peptide sequence, as expressed in the pI value (isoelectric point). The hypotheses or theories are proposed herein to help understand the present invention, and will not be used to limit the scope of the present invention.

In general, we have observed that all peptides with a pI around 4 can be loaded at about 2000 moles peptide/mole OMPC, or even higher. Moreover, using a peptide with pI < 4-5 prevents any precipitation of the conjugate. In addition, there seems to be an optimal pI value to maximize the peptide load, since further lowering of pI of the peptide sequence can cause a relative decrease of the peptide load. This probably occurs by a mechanism of electrostatic repulsion between OMPC and negatively charged peptide chains, which becomes more important as loading increases.

There seems to be a window of pI values, between 3.5-5, which favors high peptide loading. Within this range we always observe very good loading, between 2500-5000 copies. For higher pI values, the maximum attainable loading must be tuned to avoid precipitation of the conjugate. For pI values below 3.5, the maximum conjugation level of peptide chains is limited by the electrostatic repulsion effect.

The only exception to these rules is represented by peptides with either very low or very high MW. Two peptides having high pI (9.5) but low MW (1100-1330 Da) could be loaded up to 3500 moles/mole OMPC. By contrast, a high MW peptide (8000 Da) could only be loaded up to 1000 moles/mole OMPC, despite a pI of about 4. Thus, for the peptides with a low MW (<1330 Da), the adjustment of pI might not be necessary to achieve a high peptide load. In contrast, for the peptides with a low MW (>8000 Da), the adjustment of pI alone might not be sufficient to achieve a high peptide load.

We have therefore undertaken a systematic study to evaluate if modifications of the peptide sequence could have a positive impact on the load yields of the conjugates. More specifically, the underlying rationale of our approach was to modify the sequence in such a way as to lower the pI of the peptide to be conjugated to OMPC.

This strategy results in the straightforward conjugation of a plurality of peptides to a carrier such as OMPC, since no precipitation of the ensuing conjugate is observed anymore. The changes of the pI of the peptides increase the peptide load and solubility of the conjugate. In addition, manipulating peptide pI to bring it in the 3.5-5 range is generally beneficial to peptide solubility in the neutral-to-high pH buffers used for the conjugation reaction. The adjustment of the pI of the peptides therefore constitutes a general solution to the successful preparation of peptide-carrier conjugates with high-immunogenicity.

Hence, the present invention provides a method for increasing the immunogenicity of a peptide. The present invention provides a method for making a peptide-carrier conjugate. The method comprises modifying a first peptide to produce a second peptide so that the pI of the second peptide is in a favorable range or closer to the range than the pI of the first peptide, and conjugating a plurality of the second peptide to OMPC to obtain a peptide-carrier conjugate.

The peptide load, or the solubility of the conjugate, or both of them are increased by the modification. In other words, the peptide load, or the solubility of the conjugate of the second peptide and OMPC, or both of them are higher than those of the conjugate of the first peptide and OMPC. The first peptide and the second peptide are conjugated to OMPC using the same conjugation method and conditions.

The second peptide has a non-naturally occurring sequence. As used herein, "a non-naturally occurring sequence" can be the sequence containing amino acid residue, the modification at the side chain of amino residue, the modification at the termini of the peptide, which are not present in the corresponding naturally occurring sequence. As used herein, "naturally occurring sequence" is a partial or whole sequence of a naturally occurring protein or peptide.

### 2. The peptides

The peptides used in the present invention are antigens. As used herein, "antigen" refers to an agent that is capable of binding specifically to components of the immune system such as lymphocytes or antibodies.

As used herein, "immunogenicity" refers to property that allows a substance to induce a detectable immune response (humoral or cellular) when introduced into an animal. Such substances are termed "immunogens". All immunogens are antigens, but not all antigens are immunogens. Some antigens by themselves are not capable of inducing an immune response.

The immunogenicity of an antigen is determined by many factors, including molecular weight, and chemical complexity of the antigen. The antigens of less than 1000 Daltons are usually not immunogenic. The antigens of 1000 to 6000 Daltons may or may not be immunogenic. The antigens of more than 6000 Daltons are generally immunogenic.

According to a preferred embodiment of the present invention, the peptides are those with an immunogenicity that needs to be increased.

The peptides used in the present invention can be any peptides containing an epitope that are the target of the elicited immune response. As used herein, "epitope" or "antigenic determinant site" refers to individual site on surface of antigen that binds specifically with the binding site of an antibody or the receptor on the lymphocyte surface.

The peptide may have an amino acid sequence derived from a portion of a protein of any species. The species is preferably a pathogen, including bacteria, virus, and fungi. According to an embodiment of the present invention, the peptide sequence is derived from a virus protein sequence. According to a preferred embodiment, the peptide sequence is derived from protein sequences selected from *Haemophilus influenza,* hepatitis viruses A, B, or C, HIV, human papilloma virus, measles, mumps, rubella, varicella, rotavirus, *Streptococcus pneumonia* and *Staphylococcus aureus.* The peptide should include the eitope for eliciting the desired immune responses. The peptide may be either linear or cyclic peptide.

According to a preferred embodiment of the present invention, the peptide comprises a sequence selected from sequence of HA protein of Influenza virus A or B. According to another preferred embodiment of the present invention, the peptide comprises a sequence selected from HIV gp41 protein.

As used herein, the abbreviations of amino acid residues are shown as follows:

| Amino Acids | Three-Letter Abbreviations | One-Letter Abbreviations |
|---|---|---|
| Alanine | Ala | A |
| Arginine | Arg | R |
| Asparagine | Asn | N |
| Aspartate | Asp | D |
| Cysteine | Cys | C |
| Histidine | His | H |
| Isoleucine | Ile | I |
| Glutamine | Gln | Q |
| Glutamate | Glu | E |
| Glycine | Gly | G |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

According to an embodiment of the present invention, the peptide has a molecular weight at the range from 500 Da to 30000 Da. According to a preferred embodiment of the present invention, the peptide has a molecular weight at the range from 1400 Da to 7900 Da. According to a further preferred embodiment of the present invention, the peptide has a molecular weight at the range from 1800 Da to 5000 Da. According to a further more preferred embodiment of the present invention, the peptide has a molecular weight at the range from 2000 Da to 4000 Da.

The peptides used in the present invention may be linear or cyclic peptides. The linear peptides may be prepared by known solid phase peptide synthetic chemistry. (See for example E. Atherton & R. C. Sheppard, Solid Phase Peptide Synthesis. A Practical Approach. IRL Press, Oxford, England, 1989 and references cited therein; J. Jones, The Chemical Synthesis of Peptides, Oxford University Press, New York, 1991 and references cited therein; M. Bodanszky & A. Bodanszky, The Practice of Peptide Synthesis, 2nd ed., Springer-Verlag, Berlin Heidelberg, 1994 and references cited therein; Fields, G., Ed., Solid-Phase Peptide Synthesis. Methods Enzymol. 289, Academic press, New York, 1997, and references cited therein). Alternatively, the linear peptides may be prepared by fragmentation of proteins containing the peptides. The proteins may be is isolated from the pathogen, or from recombinant expression.

Cyclic peptides may be prepared by cyclization of linear peptides, for example (a) by oxidizing peptides containing at least two cysteines to generate disulfide bonded cycles; (b) by forming an amide bonded cycle; (c) by forming a thioether bonded cycle (U.S. Pat. No. 5,606,030). Processes for making the linear and cyclic peptides are described herein but this description should not be construed as being exhaustive or limiting.

### 3. The Modification of the Peptides and Adjustment of the pI

According to the present invention, a first peptide is modified to produce a second peptide so that the pI of the second peptide is in a favorable range or closer to the range than the pI of the first peptide. The second peptide is the result of the modification of the first peptide. According to the present invention, the pI of the peptide is lowered.

The pI of a peptide can be determined either with experiment such as Isoelectric focusing (IEF), or with calculation using appropriate software.

### 3.1. The modifications

In order to change their pI, the peptides of the present invention can be modified in various ways to change the charges of the peptide. The modification can be any change or changes to the peptide that result in a change in the charges of the peptide.

The peptide should be modified outside of the immunogenically active sequence, i.e., the desired epitope, thus ensuring maintenance of the immunological properties. The modification should neither involve nor interfere with the desired epitope in the peptide. Since the modifications should not impact on the immunological properties of the peptide-conjugate, changes were introduced preferably at or near the N and/or C termini of the peptide.

The modification can include the replacement, addition, or deletion of amino acid residues in the peptide. The modification can also include modification of the side chains of the residues or N-terminal amino group or C-terminal carboxylate group of the peptide. Such a modification of side chains or termini is equivalent to the replacement of an unmodified amino acid residue with a modified amino acid residue. The methods of such modifications are within the knowledge of one skilled in the art.

In the present invention, the pI of the peptides is lowered. In order to lower their pI, the peptides of the present invention can be modified in various ways to add negative charges to them. For example, adding negative charges to a peptide can be achieved by adding at least one anionic moiety to the peptide. An anionic moiety is a low molecular weight moiety having an anionic or polyanionic character at physiological pH. Alternatively, adding negative charges to a peptide can be achieved by deleting or replacing at least one cationic moiety from the peptide a low molecular weight moiety having a cationic or polycationic character at physiological pH. The anionic moiety can be an amino acid residue having anionic side chain, or an anionic group covalently linked to the side chain of an amino acid residue, or termini of the peptide.

Adding negative charges to a peptide can also be achieved by converting at least one cationic moiety into at least one anionic or neutral moiety. Examples of the conversion include acylation of N-terminal amino group of a peptide, or the γ-amino group of lysine residue.

In order to lower the pI, the modification can be addition of amino acid residues with anionic side chain to the peptide sequence at or near the N and/or C terminal of the peptide. The amino acid residues with anionic side chain can be acidic amino acid residue such as glutamate or aspartate, modified amino acid residues such as phosphoserine, or non-natural amino acid residues. The modified or added residues are preferably separated from the desired epitope sequence with a spacer composed of one or more natural or unnatural amino acids such as β-alanine or norleucine.

The modification can be at side chains of amino acid residues in the peptide sequences, or combination of any of them. The modification at the side chains includes phosphorylation or sulphation of serine, threonine, tyrosine, aspartate, or histidine, carboxylation of glutamate, acylation of lysine, or oxidation of cysteine. The modification at the side chains can be made after the synthesis of the peptides. Alternatively, the amino acid with modified side chain can be introduced into the peptide using the solid phase peptide synthetic methods. As described above, the modification is preferably made at or near the N and/or C terminal of the peptide. However, some of the modifications of the side chains, e.g., phosporylation of serine, are reversible. Thus, a modification of side chain can be made at any amino residue in the peptide, as long as the modification can be easily removed after the conjugation and the removal will not make the conjugate insoluble.

The modification can be at the N-terminal or C-terminal of the peptide sequences. For example, the modification at the termini can be acylation, such as acetylation or succinylation, of the N-terminal amino group, or carboxylation of the C terminal amide group of an amidated peptide.

The immune response raised against the peptide should cross-react with the pathogen / protein that the peptide is derived from. When used as an antigen conjugated to a carrier other than OMPC, a peptide sometimes have C terminal amidation and N-terminal acetylation, to present the peptide sequence more resembling as it would be on the native protein.

### 3.2. The favorable range of pI

According to a preferred embodiment of the present invention, the pI of the peptide is adjusted into a favorable range. According to an alternative embodiment of the present invention, the pI of the peptide is adjusted closer to, but not into the favorable range. In some situations, such as that shown in example 3, when the pI is adjusted only closer to the favorable range, the peptide load has already been improved dramatically.

According to an embodiment of the present invention, the pI of the peptide is adjusted to the range from 2 to 6. According to a preferred embodiment of the present invention, the pI of the peptide is adjusted to the range from 2.5 to 5.5. According to a further preferred embodiment of the present invention, the pI of the peptide is adjusted to the range from 3.5 to 5.

According to an embodiment of the present invention, the pI of a peptide is adjusted to the range 3.5-5 through modifications, and is then conjugated to a carrier.

The present invention provides a method suitable for different conjugation chemistries. The favorable pI range for the conjugation method other than sSMCC might be different from the range from 3.5 to 5, which is determined using sSMCC, and thereby need to be determined, following the procedure for sSMCC conjugation.

For carriers other than OMPC, such as BSA (bovine serum albumin), OVA (ovalbumin), THY (bovine thyroglobulin), KLH (keyhole limpet), conjugation of peptides can also induce precipitation of the conjugate. Similarly, a peptide sequence could be manipulated to an optimal pI value for obtaining soluble high-load conjugate of the peptide and the non-OMPC carrier. The optimal pI range for the carriers other than OMPC might be different from that for OMPC, and thereby need to be determined, as that for OMPC.

The present invention provides a method for determining the favorable pI range for conjugation. The method comprises conjugating a plurality of each of various peptides with a wide range of pI to a carrier, measuring the peptide load for each of the peptide, and determining the favorable pI range for the conjugation to achieve a high antigen load. According to an embodiment of the present invention, the various peptides are various peptides, such as those in Fig. 1.

Alternatively, a peptide can be modified in various ways to produces a series of derivatives with a range of different pI. The derivatives are conjugated to a carrier, and the favorable pI range is then determined.

### 3.3. The examples of modified peptides with adjusted pI

The adjustment of the pI of peptides results in the modified peptides. A modified peptide should have at least one difference in amino acid sequence and / or modifications from its natural origin. Examples of the modified peptides are SEQ ID NO: 2, 4, 6, 8, 9, 10 and 11. The sequences and modifications of the peptides and their derivatives are shown in the following.

| **SEQ ID NO.** | **Name** | **Sequence** | **pI** |
|---|---|---|---|
| 1 | HA₀-2 | CGPEKQTRGLFGAIAGFIENG-**NH₂** | 8.4 |
| 2 | HA₀-18 | **Ac**-CGPEKQTRGLFGAIAGFIENG**E-OH** | 4.1 |
| 3 | HA₀-9 | PEKQTRGLFGAIAGFIENGC-**NH₂** | 8.4 |
| 4 | HA₀-17 | **Suc**-EPEKQTRGLFGAIAGFIENGC-**OH** | 4 |
| 5 | PR | STMGARSMTLTVQARQLβC-**NH₂** | 12.5 |
| 6 | Succinyl-PR | **Suc**-STMGARSMTLTVQARQLβC-**NH₂** | 10.2 |
| 7 | HA₂-9 | GLFGAIAGFIENGWEGMIDG**GCGKKKK-NH₂** | 9.5 |
| 8 | HA₂-25 | GLFGAIAGFIENGWEGMVDGC**E-OH** | 3.4 |
| 9 | HA₂-20 | GLFGAIAGFIENGC**E-OH** | 3.7 |
| 10 | HA₂-22 | **Ac**-GLFGAIAGFIENGC**E-OH** | 1.1 |
| 11 | HA₂-23 | **Suc**-GLFGAIAGFIENG**CE-OH** | 1 |

| | | | |
|---|---|---|---|
| The N-terminal is either acetylated (**Ac**-), succinylated (**Suc**-), or free amine; the C-terminal is either carboxylate (-**OH**) or amide (-**NH₂**). | | | |

### 4. The Conjugation of the Peptide to a Carrier

The immunogenicity of a peptide can be increased by conjugating the peptide to a carrier, which inherently has a high immunogenicity.

### 4.1. The carriers

The carriers used in the present invention can be any protein carrier used in the art. The examples of well-known carriers include OMPC (Outer membrane protein complex of *Neisseria meningitidis),* BSA (bovine serum albumin), OVA (ovalbumin), THY (bovine thyroglobulin), KLH (keyhole limpet hemocyanin), tetanus toxoid protein, Hepatitis B virus proteins including the Surface antigen protein (HBs) and the Core Antigen protein (HBc), rotavirus capsid proteins and the L1 protein of the Human Papilloma Virus, VLP type 6, 11 or 16, etc.

According to a preferred embodiment of the present invention, the carrier is OMPC (Outer membrane protein complex of *Neisseria meningitidis*), which is a very effective immunogenic carrier. OMPC is approved by regulatory agencies worldwide as a vaccine component for polysaccharide antigens. OMPC can also be used as an immunogenic carrier for peptide antigens.

Alternatively, the carriers used in the present invention can be a carbohydrate carrier such as Dextran. Using a carbohydrate as carrier reduces the possibility of non-specific interactions because no hydrophobic or ionic sites and no protein like epitopes exist on the carrier apart from the conjugated peptide. (Bocher, et al, Journal of Immunological Methods, 208(2), 191-202 (1997).

### 4.2. The conjugation methods

The peptides and the carriers of the present invention can be conjugated using a conjugation chemistry well known in the art. According to an embodiment of the present invention, the conjugation can be achieved using sulfosuccinimidyl 4-(N-maleimidomethyl)cyclohexane-1-carboxylate (sSMCC), N-maleimidobenzoyl-N-hydroxysuccinimide ester (MBS), glutaraldehyde,1-ethyl-3-(3-dimethylaminopropyl)carbodiimide (EDCI), Bis-diazobenzidine (BDB), or N-acetyl homocysteine thiolactone (NAHT). Other conjugation methods and reagents can be found in U.S. Pat. No. 5,606,030. Different conjugation methods and reagents usually lead to different linkages between the peptides and the carrier.

According to a preferred embodiment of the present invention, the conjugation is achieved using sulfosuccinimidyl 4-(N-maleimidomethyl)cyclohexane-1-carboxylate (sSMCC), or N-maleimidobenzoyl-N-hydroxysuccinimide ester (MBS). The method using sSMCC is widely used and highly specific (See, e.g., Meyer et al. 2002, J. of Virol. 76, 2150-2158). sSMCC cross-links the SH-group of a cysteine residue to the amino group of a lysine residue on the carrier protein.

In the conjugation reaction using sSMCC, the carrier is first activated by binding the sSMCC reagent to the lysine residues of the carrier. After separation of the activated carrier from the excess reagent and the by-product, the cysteine-containing peptide is added and the link takes place by addition of the SH-group to the maleimide function of the activated carrier. The method using MBS conjugates the peptide and the carrier through a similar mechanism.

The conjugation using sSMCC is highly specific for SH-groups. Thus, cysteine residue in the peptide is essential for the conjugation. If a peptide does not have a cysteine residue, a cysteine residue should be added to the peptide, preferably at the N-terminus or C-terminus. If the desired epitope in the peptide contains a cysteine, the conjugation should be achieved with a method using neither sSMCC nor MBS. If the peptide contains more than one cysteine residue, the peptide should not be conjugated to the carrier using sSMCC or MBS, unless the excess cysteine residue can be replaced or modified.

The linkage should not interfere with the desired epitope in the peptide. The cysteine is preferably separated from the desired epitope sequence with a distance of at least one amino acid as a spacer.

According to an alternative embodiment of the present invention, the conjugation of is achieved using N-acetyl homocysteine thiolactone (NAHT). For example, thiolactones can be used to introduce a thiol functionality onto OMPC, to allow conjugation with maleimidated or Bromo-acetylated-peptides (Tolman et al. Int. J. Peptide Protein Res. 41, 1993,455-466; Conley et al. Vaccine 1994, 12, 445-451).

### 4.3. The conjugates

The present invention provides a peptide-carrier-conjugate for eliciting immune response. The conjugate is made by a process comprising modifying a first peptide to produce a second peptide so that the pI of the second peptide is in a favorable range or closer to the range than the pI of the first peptide, and conjugating a plurality of the second peptide to OMPC to obtain a peptide-carrier conjugate. The peptide load, or the solubility of the conjugate, or both of them are increased by the modification. The second peptide has a non-naturally occurring sequence.

According to an embodiment of the present invention, the peptide load is increased by more than 50%. According to a preferred embodiment of the present invention, the peptide load is increased more than 2 fold. According to a further preferred embodiment of the present invention, the peptide load is increased more than 4 fold. According to a more preferred embodiment of the present invention, the peptide load is increased more than 8 fold.

. According to a preferred embodiment of the present invention, the peptide load of the conjugate is increased to the range from 1000 to 6000 moles/mole, more preferably from 2000 to 4000 moles/mole.

### 5. The Utilities of the Conjugation Methods

The present invention can be used to produce peptide-carrier conjugates, such as peptide-OMPC conjugates, at high ratio of moles peptide/mole carrier, i.e., peptide load. The conjugate can then be used in vaccination, or the production of antibodies.

The present invention provides a conjugation method that does not depend on a specific linker or on an anionic co-conjugation scheme, but rather on engineering the peptide by straightforward modifications. By adjusting the pI of the peptide sequence, precipitation of the peptide-carrier conjugate is prevented.

Methods of the present invention do not require any fine-tuning of the reaction conditions to allow optimal loading, using small-scale experiments. Using the present invention, the activated carrier can be reacted with a large molar excess of peptide, to drive conjugation to a high peptide loading.

Furthermore, methods of the present invention are particularly suitable for large-scale process development, because they reduce the number of process steps and the number of reagents involved in the conjugation scheme.

The conjugates of the present invention can be used in different ways, including vaccine, passive immunization, and immunoassay.

### 5.1. Vaccine formulations

The conjugates of the present invention can be used as vaccines to immunize mammals including humans against infection by a pathogen such as Influenza virus or to treat humans post-infection, or to boost a pathogen-neutralizing immune response in a human afflicted with infection or disease by the pathogen.

The vaccine of the present invention can be formulated according to methods known and used in the art. Guidelines for pharmaceutical administration in general are provided in, for example, Modem Vaccinology, Ed. Kurstak, Plenum Med. Co. 1994; Remington's Pharmaceutical Sciences 18th Edition, Ed. Gennaro, Mack Publishing, 1990; and Modem Pharmaceutics 2nd Edition, Eds. Banker and Rhodes, Marcel Dekker, Inc., 1990.

Conjugates of the present invention can be prepared as various salts. Pharmaceutically acceptable salts (in the form of water- or oil-soluble or dispersible products) include conventional nontoxic salts or the quaternary ammonium salts that are formed, *e.g*., from inorganic or organic acids or bases. Examples of such salts include acid addition salts such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate; and base salts such as ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine and lysine.

It is preferred that the adjuvant is chosen as appropriate for use with the particular carrier protein used in the conjugate as well as the ionic composition of the final formulation. Consideration should also be given to whether the conjugate alone will be formulated into a vaccine or whether the conjugate will be formulated into a combination vaccine. In the latter instance one should consider the buffers, adjuvants and other formulation components that will be present in the final combination vaccine.

Aluminum based adjuvants are commonly used in the art and include Aluminum phosphate, Aluminum hydroxide, Aluminum hydroxy-phosphate and aluminum hyrdoxy-sulfate-phosphate. Trade names of adjuvants in common use include ADJUPHOS™, MERCK ALUM™ and ALHYDROGEL™. The conjugate can be bound to or co-precipitated with the adjuvant as desired and as appropriate for the particular adjuvant used.

Non-aluminum adjuvants can also be used. Non-aluminum adjuvants include QS21, Lipid-A and derivatives or variants thereof, Freund's complete or incomplete adjuvant, neutral liposomes, liposomes containing vaccine and cytokines or chemokines.

It is preferred that the vaccine be formulated with an aluminum adjuvant. In other preferred embodiments, the vaccine is formulated with both an aluminum adjuvant and QS21. It is preferable, in certain embodiments, to formulate the conjugates with immunogens from *Haemophilus Influenza,* hepatitis viruses A, B, or C, human papilloma virus, measles, mumps, rubella, varicella, rotavirus, *Streptococcus pneumonia and Staphylococus aureus.* Additionally, the vaccine of the present invention can be combined with other antigenic components of the same pathogen. In this manner a combination vaccine can be made. Combination vaccines have the advantages of increased patient comfort and lower costs of administration due to the fewer inoculations required.

When formulating combination vaccines one should be mindful of the various buffers and adjuvants used with the other immunogens. Some buffers may be appropriate for some immunogen-adjuvant pairs and not appropriate for others. In particular, one should assess the effects of phosphate levels on the various immunogen-adjuvant pairs to assure compatibility in the final formulation.

### 5.2. Vaccine administration

The vaccine of the present invention can be administered to a patient by different routes such intravenous, intraperitoneal, subcutaneous, or intramuscular. A preferred route is intramuscular. Suitable dosing regimens are preferably determined taking into account factors well known in the art including age, weight, sex and medical condition of the subject; the route of administration; the desired effect; and the particular conjugate employed (*e.g*., the peptide, the peptide loading on the carrier, etc.).

The conjugates of this invention, when used as a vaccine, are to be administered in immunologically effective amounts. An immunologically effective dose is one that stimulates the immune system of the patient to establish a level immunological memory sufficient to provide long term protection against disease caused by infection with the pathogen. The vaccine can be used in multi-dose vaccination formats. It is expected that a dose would consist of the range of 1 µg to 1.0 mg total protein. In an embodiment of the present invention the range is 0.1 mg to 1.0 mg. However, one may prefer to adjust dosage based on the amount of peptide delivered. In either case these ranges are guidelines. More precise dosages should be determined by assessing the immunogenicity of the conjugate produced so that an immunologically effective dose is delivered. The conjugate is preferably formulated with an adjuvant.

The timing of doses depend upon factors well known in the art. After the initial administration one or more booster doses may subsequently be administered to maintain antibody titers. An example of a dosing regime would be day 1,1 month, a third dose at either 4, 6 or 12 months, and additional booster doses at distant times as needed.

A patient or subject, as used herein, is a mammal and preferably a human. A patient can be of any age at which the patient is able to respond to inoculation with the present vaccine by generating an immune response. The immune response so generated can be completely or partially protective against disease and debilitating symptoms caused by infection with a pathogen such as influenza virus.

### 5.3. Other uses of the conjugates

The conjugates of the present invention can be used as immunogens to raise pathogen-neutralizing or diagnostic antibodies in a non-human mammal. The conjugate can be administered to a non-human mammal in an immunologically effective amount, to generate neutralizing immune responses against the pathogen. The mammal may be boosted with additional conjugate to elevate the immune response. Antiserum is obtained from such a mammal by bleeding the mammal, centrifuging the blood to separate the cellular component from the serum, and isolating antibody proteins from the serum if necessary, according to methods known in the art. Such antiserum or antibody preparations may be used to passively immunize humans to prevent pathogen infection, or to limit pathogen proliferation post-infection, or to treat humans afflicted with pathogen infection or disease. Such antibodies may also be used to characterize the pathogen and antigen in immunoassays.

The conjugates of the present invention can also be used directly in immunoassays, such as ELISA. The immunoassays can be used in diagnoses to detect and measure the levels of the antibodies against the antigens. The immunoassays using the conjugates have a much higher sensibility, specificity, and reproducibility than those using the antigens. (EP Patent No. 949508 A1; Bocher, M., et al, Journal of Immunological Methods, 208(2), 191-202 (1997); US Patent No. 6,114,180; Fargeas, C., et al, Journal of Clinical Microbiology, 34(2), 241-8 (1996); Carmen, E. G., et al, Journal of Virological Methods, 71, 7-16 (1998)).

### EXAMPLES

Examples are provided below to further illustrate different features of the present invention. The examples also illustrate useful methodology for practicing the invention. These examples do not limit the claimed invention.

### Example 1: HA₀-2 and HA₀-18

### 1. Peptide sequence HA₀-2

| SEQ ID NO: | Name | Peptide Sequence | pl | MW |
|---|---|---|---|---|
| 1 | HA₀-2 | **CG**PEKQTRGLFGAIAGFIENG-**NH₂** | 8.4 | 2163 |

The peptide sequence of HA₀-2 (SEQ ID NO: 1) corresponds to the region spanning the cleavage site of the Hemagglutinin protein precursor HA₀ of Influenza A sequence, H3 subtype, Hong Kong A/68. In bold there are residues, such as a glycine and a cysteine residue at the N-terminus. These are required as spacer and as cysteinyl ligand to react with a maleimide activated OMPC carrier to generate the peptide-OMPC conjugate via thioether linkage.

### 2. Peptide synthesis of HA₀-2

The peptide was synthesized by solid phase using Fmoc/t-Bu chemistry on a Pioneer Peptide Synthesizer (Applied Biosystems). The resin used was the Fmoc-Linker AM-Champion, 1% cross-linked (Biosearch Technologies, Inc.), a PEG-PS based resin derivatized with a modified Rink linker p-[(R,S)-α-[9H-Fluoren-9-yl-methoxyformamido]-2,4-dimethoxybenzyl]-phenoxyacetic acid (Rink, H. (1987) Tetrahedron Lett. 28, 3787-3789; Bernatowicz, M. S., Daniels, S. B. and Koster, H. (1989) Tetrahedron Lett. 30, 4645-4.667).

All the acylation reactions were performed for 60 min with 4-fold excess of activated amino acid over the resin free amino groups. Amino acids were activated with equimolar amounts of HBTU (2-(1H-benzotriazole-1-yl)-1,1,3,3-tetramethyluronium hexafluorophosphate) and a 2-fold molar excess of DIEA (N,N-diisopropylethylamine). The general side chain protecting group scheme was: tert-butyl for Asp, Glu, Ser, Thr and Tyr; trityl for Cys, Asn, His and Gln; 2,2,4,6,7-pentamethyldihydrobenzofuran-5-sulfonyl for Arg; tert-butoxy-carbonyl for Lys, Trp. At the end of the assembly, the dry peptide-resin was treated with 88% TFA, 5% phenol, 2% triisopropylsilane and 5% water (Sole, N. A., and Barany, G. (1992) J. Org. Chem., 57,5399-5403) for 1.5h at room temperature.

The resin was filtered and the solution was added to cold methyl-t-butyl ether in order to precipitate the peptide. After centrifugation the peptide pellets were washed with fresh cold methyl-t-butyl ether to remove the organic scavengers. The process was repeated twice. The final pellets were dried, resuspended in H₂O, 20% acetonitrile and lyophilized.

The crude peptide was purified by reverse-phase HPLC using a semi-preparative Waters RCM Delta-Pak™ C₋₁₈ cartridges (40 x 100 mm, 15 µm) using as eluents (A) 0.1% trifluoroacetic acid in water and (B) 0.1% trifluoroacetic acid in acetonitrile. We used the following gradient of B: 25%-40% over 20 min, flow rate 80 ml/min, with the peak corresponding to the product, eluting at a retention time (t_{R}) of 16'. Analytical HPLC was performed on a Ultrasphere, C₁₈ column, 25 x 4.6 mm, 5µm with the following gradient of B: 20%-50%B in 20', flow 1 ml/min. The purified peptide was characterized by electrospray mass spectrometry on a Perkin-Elmer API-100: theoretical average mw is 2163.48 Da, found 2163.6 Da.

### 3. Conjugation of peptide HA₀-2 to OMPC

Various methods of purifying OMPC from the gram-negative bacteria have been devised (Frasch et al., J. Exp. Med. 140, 87 (1974); Frasch et al., J. Exp. Med. 147, 629 (1978); Zollinger et al., U.S. Pat. No. 4,707,543 (1987); Helting et al., Acta Path. Microbiol. Scand. Sect. C. 89, 69 (1981); Helting et al., U.S. Pat. No. 4,271,147). *N. meningitidis* B improved Outer Membrane Protein Complex (iOMPC) can be obtained using techniques well known in the art such as those described by Fu, U.S. Patent No. 5,494,808.

To 2.9 mL of *Neisseria meningitidis* improved Outer Membrane Protein Complex (iOMPC) solution (6.84 mg/ml) was added 0.5 M NaHCO₃ (0.322 mL) to a final concentration of 50mM, pH 8.5. To this was added drop-wise 0.83 mL of a 20 µm solution of the heterobifunctional crosslinker sulfosuccinimidyl 4-(N-maleimidomethyl)cyclohexane-1-carboxylate (sSMCC, Pierce Chemical Co.) with a 2-fold excess (with respect to lysine residues of OMPC, 0.42 µmol lysine/mg OMPC protein). After aging the solution for 1 hour in the dark at 4°C, the pH was lowered to neutrality by adding a 1 M NaH₂PO₄ solution (46 µl). The solution was dialyzed at 4°C using 300K MWCO DispoDialyser (Spectrum Laboratories Inc., CA) with 6-buffer changes (every 2 h) of 2L, of 20mM HEPES pH 7.3 (4-(2-Hydroxyethyl)piperazine-1-ethanesulfonic acid), 2mM EDTA (Ethylenediaminetetracetic acid) to remove excess reagents. A total of 8.08 mL of activated OMPC (aOMPC) was recovered after dialysis.

A 0.7 mg/ml stock solution of the Cys-containing peptide ligand Hal₀-2, was prepared in degassed solution of 0.1 M HEPES, 2mM EDTA pH 7.3. The thiol content of the peptide solution was determined by the Ellman assay (Ellman, G. L. (1959), Arch. Biochem. Biophys., 82, 70) and showed a - SH titre of 230 µM.

To define the maximum amount of peptide ligand that could be safely incorporated on aOMPC without causing precipitation, the conjugation reaction was first followed in small-scale trials where the aOMPC was incubated with increasing amounts of peptide ligand. The maximum number of maleimide groups that can be incorporated on the OMPC is limited by the total lysine residues on the OMPC, namely 0.42 µmoles lysine/mg OMPC. If one consider an average MW of 40x10⁶ Da for OMPC, this corresponds to 16,000 lysine moles/OMPCmol. Of these only a portion can be actually activated with sSMCC up to 35%, which corresponds to a maximum peptide load attainable of about 5000 moles. Therefore aOMPC was incubated with the following molar excesses of peptide ligand per OMPC mol: 500,1000, 2000, 3000. After one hour, the samples were compared with an aOMPC sample to check for the presence of any precipitation or enhancement of turbidity.

In the case of HA₀-2 the conjugation reaction gave a soluble product only when using a molar excess up to 2000 (of moles Cys-peptide /OMPC mol) for the 1 hour incubation reaction. Above that ratio, a complete precipitation of the OMPC solution occurred.

On the basis of these observations a large-scale reaction was performed: 4 mL (9.8 mg) of aOMPC were diluted with 2.08 mL of 20 mM HEPES, 2 mM EDTA pH 7.3. To this was added 2.08 ml of the peptide stock solution, drop-wise while gently vortexing, which corresponds to 2000 molar excess of peptide moles/OMPC mol. A sample of maleimide-activated OMPC solution was retained as blank for the determination of the peptide loading of the final conjugate. The conjugation reaction mixture was allowed to age for 17h at 4°C in the dark. Any residual maleimide groups on the OMPC - were then quenched with β-mercaptoethanol to a final concentration of 15 mM (8.6 µL total volume added) for 1h at 4°C in the dark. The solution was dialyzed 4 times, 4 hour/change, with 1 L of 20 mM HEPES pH 7.3 at 4°C with 300K MWCO DispoDialyser to remove unconjugated peptide and β-mercaptoethanol.

The concentration was determined by Lowry assay (Lowry, O. H., Rosebrough, N. J., Farr, A. L. and Randall, R. J. (1951), J. Biol. Chem., 193, 265), revealing 1.0 mg/mL for the OMPC-HA₀-2. The conjugate and a aOMPC samples were hydrolyzed in evacuated, sealed glass tubes with azeotropic HCl for 70 hours at 110°C. The amino acid composition was determined by amino acid analysis. The conjugation load of peptide to OMPC protein was determined by comparing the conjugate amino acid composition with both that of the OMPC carrier and that of peptide ligand and by multiple regression, least squares analysis of the data (Shuler et al. Journal of Immunological Methods, 156, (1992) 137-149). For the conjugate OMPC and Hal₀-2, a molar ratio of peptide versus OMPC mole of 1160 was obtained.

### 4. Sequence Engineering for pI optimization

The pI of the peptide sequence of HA₀-2 is 8.4 as calculated with ProMaC (Protein Mass Calculator) software v. 1.5.3. The sequence was engineered at the C-terminus and/or the N-terminus as to lower the value of pI of the peptide below 5. In TABLE I the new peptide sequence is shown, HA₀-18 (SEQ ID NO: 2), which share with HA₀-2 the same sequence from the influenza HA₀ precursor. In bold are residues required for conjugation, spacing and pI engineering.

**TABLE I**

| SEQ ID NO: | Name | Peptide Sequence¹ | pl |
|---|---|---|---|
| 1 | HA₀-2 | **CG**PEKQTRGLFGAIAGFIENG-**NH₂** | 8.4 |
| 2 | HA₀-18 | **Ac**-CGPEKQTRGLFGAIAGFIENG**E-OH** | 4.1 |

| | | | |
|---|---|---|---|
| ¹Ac-, acetyl, CH₃-CO- | | | |

The pI lowering was accomplished by a series of modifications:
1) acetylation of the N-terminal amine group;
2) peptide C-terminal carboxylate instead of amide (assembly on a resin with an *ad-hoc* handle);
3) addition of extra acidic amino acid residues (that can be easily incorporated during stepwise peptide assembly) as for example glutamate.

### 5. Peptide synthesis of HA₀-18

The peptide was synthesized as described for HA₀-2. To produce the peptide C-terminal acid, the peptides were synthesized on a Champion PEG-PS resin (Biosearch Technologies, Inc.) that had been previously derivatized with the 4-hydroxymethylphenoxyacetic acid linker using DIPCDI/HOBt as activators. The first amino acid, Glutamate, was activated as symmetrical anhydride with DIPC (diisopropylcarbodiimide) and esterified to the resin in the presence of a catalytic amount DMAP (dimethylaminopirydine). The acetylation reaction was performed at the end of the peptide assembly by reaction with a 10-fold excess of acetic anhydride in DMF.

The crude peptide HA₀-18 was purified by reverse-phase HPLC using a semi-preparative Waters RCM Delta-Pak^{™} C₁₈ cartridges (40 x 100 mm, 15 µm) using as eluents (A) 0.1% trifluoroacetic acid in water and (B) 0.1% trifluoroacetic acid in acetonitrile. We used the following gradient of B: 30%-45% over 20 min, flow rate 80 ml/min. Analytical HPLC was performed on a Ultrasphere, C₁₈ column (Beckman), 25 x 4.6 mm, 5µm with the following gradient of B: 30%-45%B- in 20'-80% in 3', flow 1 ml/min. The purified peptides were characterized by electrospray mass spectrometry on a Perkin-Elmer API-100: theoretical average MW 2336.83 Da, found 2336 Da.

### 6. Conjugation of HA₀-18 to OMPC

The iOMPC was activated as described for HA₀-2.

A stock solution of each of the Cys-containing peptide ligand HA₀-18, was prepared in degassed solution of 0.1 M HEPES, 2mM EDTA pH 7.3. The thiol content of the peptide solutions was determined by the Ellman assay and showed a -SH titre of 200 µM.

To define the maximum amount of each peptide ligand that could be safely incorporated on aOMPC without causing precipitation, again the conjugation reaction was first followed in small-scale trials where the aOMPC was incubated with increasing amounts of each peptide ligand. Namely aOMPC was incubated with the following molar excesses of peptide ligand per OMPC mol: 1000, 2000, 3000.

After one hour, the samples were compared with a control aOMPC sample to check for presence of any precipitation or enhancement of turbidity. With the engineered sequence at lower pI, no precipitation or increase of turbidity was visible up to the highest molar excess of ligand used, 3000 moles/OMPC mol.

According to these observations, to 2 mL (4.6 mg) of aOMPC solution was added 1.68 mL of peptide stock solution (200 µM by Ellman assay, corresponding to a 3000 molar excess). The conjugation reaction mixture was allowed to age for 17h at 4°C in the dark. Any residual maleimide groups on the OMPC were then quenched with β-mercaptoethanol to a final concentration of 15 mM for 1h at 4°C in the dark. The solution was extensively dialyzed against 20 mM HEPES pH 7.3 at 4°C with 300K MWCO DispoDialyser to remove unconjugated peptide and β-mercaptoethanol. The final conjugate was analyzed by Lowry assay and amino acid analysis as described for HA₀-2. The analysis yielded a much higher level of incorporation for the lower pI peptide sequence HA₀-18 as compared to HA₀-2 in Table II:

**Table II**

| Name | Peptide Sequence¹ | pl | Pep/OMPC (molar fraction) |
|---|---|---|---|
| OMPC-HA₀-2 | **CG**PEKQTRGLFGAIAGFIENG-**NH₂** | 8.4 | 1280 |
| OMPC-HA₀-18 | **Ac-CG**PEKQTRGLFGAIAGFIENG**E-OH** | 4.1 | 2542 |

| | | | |
|---|---|---|---|
| ¹Ac-, acetyl, CH₃-CO | | | |

### Example 2: HA₀-9 and HA₀-17

### 1. Peptide sequence HA₀-9

| SEQ ID NO: | Name | Peptide Sequence | pl | MW |
|---|---|---|---|---|
| 3 | HA₀-9 | PEKQTRGLFGAIAGFIENGC-**NH₂** | 8.4 | 2107 |

The peptide sequence of HA₀-9 (SEQ ID NO: 3) corresponds to the cleavage site of the Hemagglutinin protein precursor HA₀ of Influenza A sequence, HK A/68, H3 subtype. The sequence is similar to that one of HA₀-2 in example 1, but in this case the cysteine residue needed for conjugation with the maleimide activated carrier is at the C-terminus.

### 2. Synthesis of HA₀-9

The peptide was synthesized as described for HA₀-2. The crude peptide HA₀-9 was purified by reverse-phase HPLC using a semi-preparative Waters RCM Delta-Pak^{™} C₋₁₈ cartridges (40 x 100 mm, 15 µm) using as eluents (A) 0.1 % trifluoroacetic acid in water and (B) 0.1 % trifluoroacetic acid in acetonitrile. We used the following gradient of B: 25%-4:5% over 20 min, flow rate 80 ml/min. Analytical HPLC was performed on a Ultrasphere, C₁₈ column (Beckman), 25 x 4.6 mm, 5µm with the following gradient of B: 25%-45%B- in 20'-80% in 3', flow 1 ml/min. The purified peptides were characterized by electrospray mass spectrometry on a Perkin-Elmer API-100: theoretical average MW 2106.43 Da, found 2107 Da.

### 3. Conjugation attempt of HA₀-9 to aOMPC

The iOMPC was activated as described in example 1.

A stock solution of HA₀-9, was prepared in degassed solution of 0.1 M HEPES, 2mM EDTA pH 7.3. The thiol content of the peptide solutions was determined by the Ellman assay and showed a -SH titre of 250 µM.

To define the maximum amount of peptide ligand that could be safely incorporated on aOMPC without causing precipitation, the conjugation reaction was first followed in small-scale trials where the aOMPC was incubated with increasing amounts of HA₀-9. Namely aOMPC was incubated with the following molar excesses of peptide ligand per OMPC mol: 200, 500, 1000, 2000. After one hour, the samples were compared with an aOMPC sample to check for the presence of any precipitation or enhancement of turbidity. In this case the conjugation reaction did not give any soluble product even at the lowest molar excess tested (of moles Cys-peptide per OMPC mol). On the basis of these observations, no large scale coniugation was performed for this peptide.

### 4. HA₀-17: sequence Engineering of H₀-9 for pI optimization

The pI of the peptide sequence of HA₀-9 is 8.4 as calculated with ProMaC (Protein Mass Calculator) software v. 1.5.3. The sequence was engineered as to lower the value of pI of the peptide to the value of 4. The modifications include a Cys terminal carboxylate instead of amide, addition of a Glutamate and a succinyl at the N-terminus (Table III). The engineered peptide is HA₀-17 (SEQ ID NO: 4).

### 5. Peptide synthesis of HA₀-17

To produce the peptide C-terminal acid, the synthesis was performed on a Champion PEG-PS resin (Biosearch Technologies, Inc.) that had been previously derivatized with the 4-hydroxymethylphenoxyacetic acid linker using DIPCDI/HOBt as activators. The first amino acid, Glutamate, was activated as symmetrical anhydride with DIPC (diisopropylcarbodiimide) and esterified to the resin in the presence of a catalytic amount DMAP (dimethylaminopirydine). The assembly was performed as described for HA₀-2. The succinylation reaction was performed at the end of the peptide assembly by reaction with a 10-fold excess of succinic anhydride in DMF.

The crude peptide HA₀-17 was purified by reverse-phase HPLC using a semi-preparative Waters RCM Delta-Pak^{™} C₋₁₈ cartridges (40 x 100 mm, 15 µm) using as eluents (A) 0.1% trifluoroacetic acid in water and (B) 0.1 % trifluoroacetic acid in acetonitrile. We used the following gradient of B: 30%-45% over 20 min, flow rate 80 ml/min. Analytical HPLC was performed on a Ultrasphere, C₁₈ column (Beckman), 25 x 4.6 mm, 5µm with the following gradient of B: 30%-45%- in 20'-80% in 3', flow 1 ml/min. The purified peptide was characterized by electrospray mass spectrometry on a Perkin-Elmer API-100: theoretical average MW 2337.62 Da, found 2336,8 Da.

### 6. Conjugation of HA₀-17 to aOMPC

The iOMPC was activated as described in example 1.

A stock solution of HA₀-17, was prepared in degassed solution of 0.1 M HEPES, 2mM EDTA pH 7.3. The thiol content of the peptide solutions was determined by the Ellman assay and showed a -SH titre of 200 µM.

To define the maximum amount of peptide ligand that could be safely incorporated on aOMPC without causing precipitation, the conjugation reaction was first followed in small-scale trials where the aOMPC was incubated with increasing amounts of HA₀-17. Namely aOMPC was incubated with the following molar excesses of peptide ligand per OMPC mol: 1000, 2000, 3500. After one hour, the samples were compared with an aOMPC sample to check for the presence of any precipitation or enhancement of turbidity. With the engineered sequence at lower pI, no precipitation or increase of turbidity was visible up to the highest molar excess of ligand used, 3500 moles/OMPC mol.

On the basis of these observations, a large-scale reaction was performed on 3 mg (0.94 mL) of aOMPC. To this solution, 1.334 mL of the peptide stock solution were added drop-wise while gently vortexing, which corresponds to 3500 molar excess of peptide moles/OMPC mole. The conjugation reaction mixture was allowed to age for 17h at 4°C in the dark. Any unreacted maleimide groups on the OMPC were then reacted with β-mercaptoethanol to a final concentration of 15 mM for 1h at 4°C in the dark. The solution was extensively dialyzed against 20 mM HEPES pH 7.3 at 4°C with 300K MWCO DispoDialyser to remove unconjugated peptide and β-mercaptoethanol. The final conjugate was analyzed by Lowry assay and amino acid analysis as described for HA₀-2. The analysis yielded a level of incorporation of 1860 moles peptide/mol OMPC, while no conjugation was possible for the parent HA₀-9 peptide sequence as shown in Table III:

**Table III**

| Name | Peptide Sequence¹ | pl | Pep/OMPC (molar fraction) |
|---|---|---|---|
| HA₀-9-OMPC | PEKQTRGLFGAIAGFIENG**C-NH₂** | 8.4 | 0 |
| HA₀-17-OMPC | **Suc-E**PEKQTRGLFGAIAGFIENG**C-OH** | 4 | 1860 |

| | | | |
|---|---|---|---|
| ¹Suc-, succinyl, HOOC-(CH₂)₂-CO- | | | |

### Example 3: PR and PR and Succinyl-PR

### 1. Peptide sequence PR

| SEQ ID NO: | Name | Peptide sequence¹ | pl | MW |
|---|---|---|---|---|
| 5 | PR | STMGARSMTLTVQARQLβ**C-NH₂** | 12.5 | 2023 |

| | | | | |
|---|---|---|---|---|
| ¹β, β-alanine | | | | |

The sequence of PR (SEQ ID NO: 5) is a portion of the HTV-1 gp41 protein, spanning the so-called polar region of gp41. The portion in bold are residues, required as a spacer such as beta alanine (βAla) and for conjugation.

### 2. Synthesis of PR

The peptide was synthesized as described for HA₀-2. The crude peptide PR was purified by reverse-phase HPLC using a semi-preparative Waters RCM Delta-Pak^{™} C₋₁₈ cartridges (40 x 100 mm, 15 µm) using as eluents (A) 0.1% trifluoroacetic acid in water and (B) 0.1% trifluoroacetic acid in acetonitrile. We used the following gradient of B: 10%-30% over 20 min, flow rate 80 ml/min. Analytical HPLC was performed on a Ultrasphere, C₁₈ column (Beckman), 25 x 4.6 mm, 5µm with the following gradient of B: 15%-30%- in 20'-80% in 3', flow 1 ml/min. The purified peptides were characterized by electrospray mass spectrometry on a Perkin-Elmer API-100: theoretical average MW 2023,42 Da, found 2023,2 Da.

### 3. Conjugation of PR to aOMPC

The iOMPC was activated as described in example 1.

A stock solution of PR, was prepared in degassed solution of 0.1 M HEPES, 2mM EDTA pH 7.3. The thiol content of the peptide solutions was determined by the Ellman assay and showed a -SH titre of 250 µM.

To define the maximum amount of peptide ligand that could be safely incorporated on aOMPC without causing precipitation, the conjugation reaction was first followed in small-scale trials where the aOMPC was incubated with increasing amounts of PR. Namely aOMPC was incubated with the following molar excesses of peptide ligand per OMPC mol: 300, 500, 1000, 2000, 4000. After one hour, the samples were compared with an aOMPC sample to check for the presence of any precipitation or enhancement of turbidity. In this case the conjugation reaction gave a soluble product only when using a molar excess of 300 (of moles Cys-peptide per OMPC mol) for the 1 hour incubation reaction. Above that ratio, a complete precipitation of the OMPC solution occurred.

On the basis of these observations a large-scale reaction was performed on 3 mg (0.94 mL) of aOMPC. To this solution was added 0.79 mL of 20 mM HEPES pH 7.3, 2 mM EDTA, and 0.094 mL (300 molar excess of peptide moles/OMPC mole) of the peptide stock solution drop-wise while gently vortexing. The conjugation reaction mixture was allowed to age for 17h at 4°C in the dark. Any residual maleimide groups on the OMPC were then quenched with β-mercaptoethanol to a final concentration of 15 mM for 1h at 4°C in the dark. The solution was extensively dialyzed against 20 mM HEPES pH 7.3 at 4°C with 300K MWCO DispoDialyser to remove unconjugated peptide and β-mercaptoethanol. The final conjugate was analyzed by Lowry assay and amino acid analysis as described for HA₀-2. The analysis yielded a low level of incorporation of 200 moles peptide/mol OMPC.

### 4. Succinyl-PR: sequence engineering of PR

The pI of the peptide sequence of PR is very high 12.5, as calculated with ProMaC (Protein Mass Calculator) software v. 1.5.3. The peptide sequence was modified by succinylation of the N-terminal free amino group resulting in the peptide sequence Succinyl-PR (SEQ ID NO: 6) with a pI of 10.2 shown in Table IV. Thus, the pI of Succinyl-PR is still not in the favorable range (from 3.5 to 5), although it is closer to the favorable range than that of PR.

### 5. Peptide synthesis of Succinyl-PR

The assembly was performed as described for HA₀-2. The succinylation reaction was performed at the end of the peptide assembly by reaction with a 10-fold excess of succinic anhydride in DMF. The crude peptide PR was purified by reverse-phase HPLC using a semi-preparative Waters RCM Delta-Pak^{™} C₋₁₈ cartridges (40 x 100 mm, 15 µm) using as eluents (A) 0.1% trifluoroacetic acid in water and (B) 0.1% trifluoroacetic acid in acetonitrile. We used the following gradient of B: 10%-30% over 20 min, flow rate 80 ml/min. Analytical HPLC was performed on a Ultrasphere, C₁₈ column (Beckman), 25 x 4.6 mm, 5µm with the following gradient of B: 10%-30%B in 20'-80% in 3', flow 1 ml/min. The purified peptide was characterized by electrospray mass spectrometry on a Perkin-Elmer API-100: theoretical average MW 2124,51 Da, found 2123,2 Da.

### 6. Conjugation of Succinyl PR to aOMPC

The iOMPC was activated as described in example 1.

A stock solution of Succinyl-PR, was prepared in degassed solution of 0.1 M HEPES, 2mM EDTA pH 7.3. The thiol content of the peptide solutions was 235 µM.

To define the maximum amount of peptide ligand that could be safely incorporated on aOMPC without causing precipitation, the conjugation reaction was followed in small-scale trials where the aOMPC was incubated with increasing amounts of Succinyl-PR. Namely aOMPC was incubated with the following molar excesses of peptide ligand per OMPC mol: 500, 1000, 2000, 4000. After one hour, the samples were compared with an aOMPC sample to check for the presence of any precipitation or enhancement of turbidity. In this case the conjugation reaction gave a slight presence of turbidity at the highest 4000 molar excess tested for the 1 hour incubation reaction.

On the basis of these observations a large-scale reaction was performed on 2.95 mg (1.2 ml) of aOMPC which was first diluted with 0.312 mL of 20 mM HEPES, 2 mM EDTA pH 7.3. To this solution of aOMPC, 0.936 mL of the peptide stock solution were added drop-wise while gently vortexing which corresponds to 3000 molar excess of peptide moles/OMPC mole. The conjugation reaction mixture was allowed to age for 17h at 4°C in the dark. Any unreacted maleimide-groups on the OMPC were then reacted with β-mercaptoethanol to a final concentration of 15 mM for 1h at 4°C in the dark. The solution was extensively dialyzed against 20 mM HEPES pH 7.3 at 4°C with 300K MWCO DispoDialyser to remove unconjugated peptide and β-mercaptoethanol. The final conjugate was analyzed by Lowry assay and amino acid analysis as described for HA₀-2. The analysis yielded a level of incorporation of 1360 moles peptide/mol OMPC, much higher with respect to the parent PR peptide sequence as shown in Table IV:

**Table IV**

| Name | Peptide sequence¹ | pl | Pep/OMPC (molar fraction) |
|---|---|---|---|
| PR-OMPC | STMGARSMTLTVQARQLβ**C-NH₂** | 12.5 | 200 |
| Succinyl-PR-OMPC | **Suc**-STMGARSMTLTVQARQLβ**C-NH₂** | 10.2 | 1360 |

| | | | |
|---|---|---|---|
| ¹Suc-, succinyl, HOOC-(CH₂)₂-CO-, β, β-alanine | | | |

Hence, even a modification that only adjusts the pI closer to the favorable range may be sufficient to improve the peptide load dramatically.

### Example 4: HA₂-9 and HA₂-25

### 1. Peptide sequence HA₂-9

| SEQ ID NO: | Name | Peptide Sequence | MW |
|---|---|---|---|
| 7 | HA₂-9 | GLFGAIAGFIENGWEGMIDG**GCGKKKK-NH2** | 2783 |

The peptide sequence of HA₂-9 (SEQ ID NO: 7) corresponds to the fusion peptide region of the Hemagglutinin protein HA₂ of Influenza A sequence, H3 subtype, Hong Kong A/68. The sequence contains residues (in bold) such as lysines at the C-terminus to increase solubility, a Cysteine for conjugation with maleimide activated OMPC and a Gly as a spacer.

### 2. Synthesis of HA₂-9

The peptide was synthesized as described for HA₀-2. The crude peptide HA₂-9 was purified by reverse-phase HPLC using a semi-preparative Waters RCM Delta-Pak^{™} C₋₁₈ cartridges (40 x 100 mm, 15 µm) using as eluents (A) 0.1% trifluoroacetic acid in water and (B) 0.1% trifluoroacetic acid in acetonitrile. We used the following gradient of B: 30%-55% over 20 min, flow rate 80 ml/min. Analytical HPLC was performed on a Ultrasphere, C₁₈ column (Beckman), 25 x 4.6 mm, 5µm with the following gradient of B: 30%-55%B- in 20'-80% in 3', flow 1 ml/min. The purified peptides were characterized by electrospray mass spectrometry on a Perkin-Elmer API-100: theoretical average MW 2782.28 Da, found 2783 Da.

### 3. Conjugation of HA₂-9 to aOMPC

The iOMPC was activated as described in example 1.

A stock solution of HA₂-9, was prepared in degassed solution of 0.1 M HEPES, 2mM EDTA pH 7.3. The thiol content of the peptide solutions was determined by the Ellman assay, showing a -SH titre of 200 µM.

To define the maximum amount of peptide ligand that could be safely incorporated on aOMPC without causing precipitation, the conjugation reaction was first followed in small-scale trials where the aOMPC was incubated with increasing amounts of HA₂-9. Namely, aOMPC was incubated with the following molar excesses of peptide ligand per OMPC mol: 200, 500, 1000, 2000, 4000, 6000. After one hour, the samples were compared with an aOMPC sample to check for the presence of any precipitation or enhancement of turbidity. In this case the conjugation reaction gave a soluble product only when using a molar excess of 200 (moles Cys-peptide per OMPC mol) for the 1 hour incubation reaction. Above that ratio, a complete precipitation of the OMPC solution occurred.

On the basis of these observations a large-scale reaction was performed on 3.4 mg (0.8 mL) of aOMPC. To this solution was added 0.662 mL of 20 mM HEPES, 2 mM EDTA pH 7.3. and 0.074 mL of the peptide stock solution drop-wise while gently vortexing which corresponds to 200 molar excess of peptide moles/OMPC mole. The conjugation reaction mixture was allowed to age for 17h at 4°C in the dark. Any unreacted maleimide groups on the OMPC were then reacted with β-mercaptoethanol to a final concentration of 15 mM for 1h at 4°C in the dark. The solution was extensively dialyzed against 20 mM HEPES pH 7.3 at 4°C with 300K MWCO DispoDialyser to remove unconjugated peptide and β-mercaptoethanol. The final conjugate was analyzed by Lowry assay and amino acid analysis as described for HA₀-2. The analysis yielded a low level of incorporation of 544 moles peptide/mol OMPC (overestimated level of incorporation 500 vs 200 within the error of the analysis).

### 4. HA₂-25: sequence Engineering of HA₂-9 for pI optimization

The pI of the peptide sequence of HA₂-9 is 9.5, as calculated with ProMaC (Protein Mass Calculator) software v. 1.5.3. The sequence was modified at the C-terminus as to lower the value of pI of the peptide to 3.4. The modifications comprise deletion of the basic tail and incorporation of a glutamate as C-terminal residue, resulting in HA₂-25 (SEQ ID NO: 8).

**TABLE V**

| SEQ ID NO: | Name | Peptide Sequence | pl |
|---|---|---|---|
| 7 | HA₂-9 | GLFGAIAGFIENGWEGMIDG**GCGKKKK-NH2** | 9.5 |
| 8 | HA₂-25 | GLFGAIAGFIENGWEGMVDG**CE-OH** | 3.4 |

### 5. Peptide synthesis of HA₂-25

To produce the peptide C-terminal acid, the peptide was synthesized on a Champion PEG-PS resin (Biosearch Technologies, Inc.) that had been previously derivatized with the 4-hydroxymethylphenoxyacetic acid linker using DIPCDI/HOBt as activators. The first amino acid, Glutamate, was activated as symmetrical anhydride with DIPC (diisopropylcarbodiimide) and esterified to the resin in the presence of a catalytic amount DMAP (dimethylaminopirydine). The assembly was performed as described for HA₀-2.

The crude peptide HA₂-25 was purified by reverse-phase HPLC using a semi-preparative Waters RCM Delta-Pak^{™}C₄ cartridges (40 x 100 mm, 15 µm) using as eluents (A) 0.1% trifluoroacetic acid in water and (B) 0.1% trifluoroacetic acid in acetonitrile. We used the following gradient of B: 40%-40%(5')-60%(20'), flow rate 80 ml/min. Analytical HPLC was performed on a Phenomenex, Jupiter C₄ column, 15 x 4.6 mm, 5µm with the following gradient of B: 35%-55%- in 20'-80% in 3', flow 1 ml/min. The purified peptide was characterized by electrospray mass spectrometry on a Perkin-Elmer API-100: theoretical average MW 2271,55 Da, found 2271,2 Da.

### 6. Conjugation of HA₂-25 to aOMPC

The iOMPC was activated as described in example 1.

A solution of HA₂-25, was prepared in degassed solution of 0.1 M HEPES, 2mM EDTA pH 7.3. The thiol content of the peptide solutions was determined by the Ellman assay and showed a -SH titre of 250 µM.

To define the maximum amount of peptide ligand that could be safely incorporated on aOMPC without causing precipitation, the conjugation reaction was first followed in small-scale trials where the aOMPC was incubated with increasing amounts of HA₂-25. Namely, aOMPC was incubated with the following molar excesses of peptide ligand per OMPC mol: 500, 1000, 2000, 4000, 6000. After one hour, the samples were compared with an aOMPC sample to check for the presence of any precipitation or enhancement of turbidity. With the engineered sequence at lower pI, no precipitation or increase of turbidity was visible up to the highest molar excess of ligand used, 6000 moles/OMPC mol.

According to these observations the large-scale reaction was performed on 6.3 mg (2.57 ml) of aOMPC. To this was added. 3.85 mL of the peptide stock solution drop-wise while gently vortexing which corresponds to 6000 molar excess of peptide moles/OMPC mole. The conjugation reaction mixture was allowed to age for 17h at 4°C in the dark. Any unreacted maleimide groups on the OMPC were then reacted with β-mercaptoethanol to a final concentration of 15 mM for 1h at 4°C in the dark. The solution was extensively dialyzed against 20 mM HEPES pH 7.3 at 4°C with 300K MWCO DispoDialyser to remove unconjugated peptide and β-mercaptoethanol. The final conjugate was analyzed by Lowry assay and amino acid analysis as described for HA₀-2. The analysis yielded a level of incorporation for HA₂-25 of 2436 moles peptide/mol OMPC, much higher with respect to the parent HA₂-9 peptide sequence as shown in Table VI:

**Table VI**

| Name | Peptide sequence | pl | Pep/OMPC (molar fraction) |
|---|---|---|---|
| HN₂-9-OMPC | GLFGAIAGFIENGWEGMIDG**GCGKKKK-NH2** | 9.5 | 544 |
| HA₂-25-OMPC | GLFGAIAGFIENGWEGMVDG**CE-OH** | 3.4 | 2436 |

Examples 1-4 show that modifications of the peptide sequences at either terminus, to bring the pI below a certain value, result in a higher level of conjugation of the pI engineered sequences on the OMPC carrier.

### Example 5: HA₂-20, HA₂-22 and HA₂-23

### 1. Peptide sequences of HA₂-20, HA₂-22 and HA₂-23

| SEQ ID NO: | Name | Peptide Sequence | pl |
|---|---|---|---|
| 9 | HA₂-20 | GLFGAIAGFIENG**CE-OH** | 3.7 |
| 10 | HA₂-22 | **Ac**-GLFGAIAGFIENG**CE-OH** | 1.1 |
| 11 | HA₂-23 | **Suc**-GLFGAIAGFIENG**CE-OH** | 1 |

The sequence of the three peptides corresponds to the fusion peptide region of the Hemagglutinin protein HA₂ of Influenza A sequence, H3 subtype. The sequence contain residues (in bold) such as a Cysteine for conjugation with maleimide activated OMPC, and a Glutamate at the C terminus to lower the pI of the peptide sequence. The N-terminus differs in the three peptides, with a free amino group for HA₂-20 (SEQ ID NO: 9), acetyl for the HA₂-22 (SEQ ID NO: 10) and sucinyl-group for HA₂-23 (SEQ ID NO: 11).

### 2. Synthesis of HA₂-20, HA₂-22 and HA₂-33

The peptides were synthesized as described for HA₀-2. At the end of assembly HA2-22 was acetylated with a 10-fold excess of acetic anhydride in DMF and HA2-23 was succinylated with a 10-fold excess of succinic anhydride. The crude peptide HA₂-20 was purified by reverse-phase HPLC using a semi-preparative Waters RCM Delta-Pak^{™} C₋₁₈ cartridges (40 x 100 mm, 15 µm) using as eluents (A) 0.1% trifluoroacetic acid in water and (B) 0.1% trifluoroacetic acid in acetonitrile. We used the following gradient of B: 35%-35%(5')-50% over 20 min, flow rate 80 ml/min. Analytical HPLC was performed on a Ultrasphere, C₁₈ column (Beckman), 25 x 4.6 mm, 5µm with the following gradient of B: 35%-50%B- in 20'-80% in 3', flow 1 ml/min. The purified peptide was characterized by electrospray mass spectrometry on a Perkin-Elmer API-100: theoretical average MW 1496,70 Da, found 1496,8 Da.

The crude peptide HA₂-22 was purified by reverse-phase HPLC using a semi-preparative Waters RCM Delta-Pak^{™} C-18 cartridges (40 x 100 mm, 15 µm) using as eluents (A) 0.1% trifluoroacetic acid in water and (B) 0.1% trifluoroacetic acid in acetonitrile. We used the following gradient of B: 40%-40%(5')-55% over 20 min, flow rate 80 ml/min. Analytical HPLC was performed on a Ultrasphere, C₁₈ column (Beckman), 25 x 4.6 mm, 5µm with the following gradient of B: 40%-55%B- in 20'-80% in 3', flow 1 ml/min. The purified peptide was characterized by electrospray mass spectrometry on a Perkin-Elmer API-100: theoretical average MW 1539,74 Da, found 1539,0 Da.

The crude peptide HA₂-23 was purified by reverse-phase HPLC using a semi-preparative Waters RCM Delta-Pak^{™} C₋₁₈ cartridges (40 x 100 mm, 15 µm) using as eluents (A) 0.1% trifluoroacetic acid in water and (B) 0.1% trifluoroacetic acid in acetonitrile. We used the following gradient of B: 40%-40%(5')-55% over 20 min, flow rate 80 ml/min. Analytical HPLC was performed on a Ultrasphere, C₁₈ column (Beckman), 25 x 4.6 mm, 5µm with the following gradient of B: 40%-55%B- in 20'-80% in 3', flow 1 ml/min. The purified peptide was characterized by electrospray mass spectrometry on a Perkin-Elmer API-100: theoretical average MW 1597,78 Da, found 1596,8 Da.

### 3. Conjugation of HA₂-20, HA₂-22 and HA₂-23 to aOMPC

The iOMPC was activated as described in example 1.

A solution of the three peptides was prepared in degassed solution of 0.1 M HEPES, 2mM EDTA pH 7.3. The thiol content of the peptide solutions were determined by the Ellman assay and showing a -SH titre of: 333 µm for HA₂-20, 325 µM for HA₂-22 and 313 µM for HA₂-23.

To define the maximum amount of the three peptide ligands that could be safely incorporated on aOMPC without causing precipitation, the conjugation reaction was first followed in small-scale trials where the aOMPC was incubated with increasing amounts of each of the three peptides: HA₂-20,HA₂-22 and HA₂-23. Namely, aOMPC was incubated with the following molar excesses of peptide ligand per OMPC mol: 500, 1000, 2000, 4000, 6000. After one hour, the samples were compared with an aOMPC sample to check for the presence of any precipitation or enhancement of turbidity. For all the three peptides no precipitation or increase of turbidity was visible up to the highest molar excess of ligand used, 6000 moles/OMPC mol.

According to these observations the large-scale reaction was performed on 6.3 mg (2.57 ml) of aOMPC. To this was added. 3.85 mL of the peptide stock solution drop-wise while gently vortexing which corresponds to 6000 molar excess of peptide moles/OMPC mole. The conjugation reaction mixture was allowed to age for 17h at 4°C in the dark. Any unreacted maleimide groups on the OMPC were then reacted with β-mercaptoethanol to a final concentration of 15 mM for 1h at 4°C in the dark. The solution was extensively dialyzed against 20 mM HEPES, pH 7.3 at 4°C with 300K MWCO DispoDialyser to remove unconjugated peptide and β-mercaptoethanol. The final conjugates were analyzed by Lowry assay and amino acid analysis as described for HA₀-2. The analysis yielded a level of incorporation higher for HA₂-20 at a pI of 3.7 and lower for HA₂-22 and HA₂-23 (pH ca. 1) as shown in Table VII:

**Table VII**

| Short Name | Peptide Sequence¹ | pl | Pep/OMPC (molar fraction) |
|---|---|---|---|
| HA₂-20-OMPC | GLFGAIAGFIENG**CE-OH** | 3.7 | 2647 |
| HA₂-22-OMPC | **Ac**-GLFGAIAGFIENG**CE-OH** | 1.1 | 1785 |
| HA₂-23-OMPC | **Suc**-GLFGAIAGFIENG**CE-OH** | 1 | 1616 |

| | | | |
|---|---|---|---|
| ¹Ac-, acetyl, CH₃-CO, Suc-, succinyl, HOOC-(CH₂)₂-CO- | | | |

This example highlights that there seems to be an optimal pI value to maximize the peptide load, since further lowering of pI of the peptide sequence can cause a relative decrease of the peptide load.

### Example 6: The Optimal pI Range for the Conjugation of Peptides to OMPC

To have an estimation on how general the correlation is, between the actual pI value of peptide sequences and the loads on the OMPC carrier, we plotted the molar ratio pep moles/OMPC mol as a function of pI for a number of peptide sequences for which we performed conjugation to OMPC (Fig. 1).

The pI lowering was accomplished by a series of modifications:
1) acetylation or succinylation of the N-terminal amine group;
2) peptide C-terminal carboxylate instead of amide (assembly on a resin with an *ad-hoc* handle);
3) addition of extra acidic amino acid residues (that can be easily incorporated during stepwise peptide assembly) as for example glutamate.

There seems to be a window of pI values, between 3.5-5, which favors high peptide loading within this range we always observe very good loading, between 2500-5000 copies. For higher pI values, the maximum attainable loading must be tuned to avoid precipitation of the conjugate. For pI values below 3.5, the maximum conjugation level of peptide chains is limited by the electrostatic repulsion effect.

The only observed exception to these rules is represented by peptides with either very low or very high MW. Two peptides having high pI (9.5) but low MW (1100-1330 Da) could be loaded up to 3500 moles/mole OMPC. By contrast, a high MW peptide (8000 Da) could only be loaded up to 1000 moles/mole OMPC, despite a pI of about 4.

## Claims

1. A method for making a peptide-carrier conjugate comprising:
a) modifying a first peptide to produce a second peptide that has a lower isoelectric point (pI) than the first peptide; and
b) conjugating a plurality of the second peptide to an outer membrane protein complex (OMPC) carrier protein to obtain a peptide-carrier conjugate,
wherein at least one of peptide load and solubility of the conjugate consisting of the plurality of the second peptide and OMPC carrier protein is increased as compared to a conjugate of a plurality of the first peptide and OMPC carrier protein and wherein the second peptide has a non-naturally occurring sequence.

2. The method of claim 1 wherein the second peptide has a pI that is lower than 6.

3. The method of claim 1 wherein the second peptide has a pI that is between 2 and 6.

4. The method of claim 1 wherein the second peptide has a pI that is between 3.5 and 5.

5. The method of claim 1 wherein the first peptide has an amino acid sequence selected from a pathogen.

6. The method of claim 5 wherein the pathogen is selected from the group consisting of Haemophilus influenza, hepatitis viruses A, B, or C, HIV, human papilloma virus, measles, mumps, rubella, varicella, rotavirus, Streptococcus pneumonia and Staphylococcus aureus.

7. The method of claim 1 wherein the first peptide has an amino acid sequence selected from the group consisting of:
a) HA protein of Influenza virus A or B; and
b) gp41 protein of HIV.

8. The method of claim 1 wherein the first peptide is modified by adding at least one amino acid with an anionic side chain.

9. The method of claim 8 wherein the at least one amino acid with an anionic side chain is selected from the group consisting of Glutamate and Aspartate.

10. The method of claim 1 wherein the first peptide is modified at a side chain of an amino acid residue.

11. The method of claim 10 wherein the side chain is modified in a manner selected from the group consisting of
a) phosphorylation of serine, threonine, tyrosine, aspartate, or histidine;
b) sulphation of serine, threonine, tyrosine, aspartate, or histidine;
c) carboxylation of glutamate;
d) acylation of lysine; and
e) oxidation of cysteine.

12. The method of claim 1 wherein the first peptide is modified at least one of its N-terminal or C-terminal residues.

13. The method of claim 12 wherein the first peptide is an amidated peptide and is modified with carboxylation of the C-terminal residue.

14. The method of claim 12 wherein the first peptide is modified with acylation of the N-terminal amino group.

15. The method of claim 14 wherein the acylation is acetylation or succinylation.

16. The method of claim 1 wherein the plurality of the second peptide are conjugated to the carrier using an agent selected from the group consisting of sulfosuccinimidyl 4-(N maleimidomethyl)cyclohexane-1-carboxylate (sSMCC), N-maleimidobenzoyl-N-hydroxysuccinimide ester (MBS), glutaraldehyde, 1-methyl-3-(3-dimethylaminopropyl)carbodiimide (EDCI), Bis diazobenzidine (BDB), or N-acetyl homocysteine thiolactone (NAHT), N-maleimidobenzoyl-N hydroxysuccinimide ester (MB S), glutaraldehyde, 1-ethyl-3-(3-dimethylaminopropyl)carbodiimide (EDCI), Bis-diazobenzidine (BDB), and N-acetyl homocysteine thiolactone (NAHT).

17. The method of claim 16 wherein the agent is sSMCC.

18. The method of claim 1 wherein the first peptide has a molecular weight at the range from 500 Da to 30000 Da.

19. The method of claim 1 wherein the first peptide has a molecular weight at the range from 1400 Da to 7900 Da.

20. The method of any of claims 1-19 wherein the peptide load is increased.

21. The method of claim 1 wherein the peptide load is increased to more than 500 moles of peptide/mole of carrier protein.

22. The method of claim 1 wherein the peptide load is increased to more than 1000 moles of peptide/mole of carrier protein.

23. A method for determining a favorable pI range for conjugation of a peptide to an OMPC carrier protein comprising:
a) conjugating a plurality of the peptide to the carrier, each of the peptides having been modified such that there is a plurality of different pIs; and
b) measuring the peptide load for each of the peptides,
wherein the favorable pI range is determined by the pIs of the peptides having the most favorable peptide load.

24. The method of claim 23 wherein the favorable pI range is determined by the pIs of those peptides having a peptide load of more than 500 moles of peptide/mole of carrier protein.

25. The method of claim 23 wherein the favorable pI range is determined by the pIs of those peptides having a peptide load of more than 1000 moles of peptide/mole of carrier protein.

## Patentansprüche

1. Verfahren zur Herstellung eines Peptid-Träger-Konjugats, umfassend:
a) Modifizieren eines ersten Peptids, um ein zweites Peptid herzustellen, das einen niedrigeren isoelektrischen Punkt (pl) als das erste Peptid besitzt, und
b) Konjugieren einer Vielzahl des zweiten Peptids an ein Außenmembran-Proteinkomplex (OMPC)-Trägerprotein, um ein Peptid-Träger-Konjugat zu erhalten,
wobei wenigstens eines von Peptidbeladung und Löslichkeit des Konjugats, das aus der Vielzahl des zweiten Peptids und OMPC-Trägerprotein besteht, im Vergleich zu einem Konjugat aus einer Vielzahl des ersten Peptids und OMPC-Trägerprotein erhöht ist und wobei das zweite Peptid eine nicht natürlich vorkommende Sequenz besitzt.

2. Verfahren nach Anspruch 1, wobei das zweite Peptid einen pl besitzt, der niedriger als 6 ist.

3. Verfahren nach Anspruch 1, wobei das zweite Peptid einen pl besitzt, der zwischen 2 und 6 liegt.

4. Verfahren nach Anspruch 1, wobei das zweite Peptid einen pl besitzt, der zwischen 3,5 und 5 liegt.

5. Verfahren nach Anspruch 1, wobei das erste Peptid eine Aminosäuresequenz besitzt, die aus einem Pathogen ausgewählt wird.

6. Verfahren nach Anspruch 5, wobei das Pathogen aus der Gruppe, bestehend aus Haemophilus influenza, Hepatitis-Viren A, B oder C, HIV, humanem Papilloma-Virus, Masern, Mumps, Rubella, Varicella, Rotavirus, Streptococcus pneumonia und Staphylococcus aureus, ausgewählt wird.

7. Verfahren nach Anspruch 1, wobei das erste Peptid eine Aminosäuresequenz besitzt, die aus der Gruppe, bestehend aus:
a) HA-Protein von Influenza-Virus A oder B und
b) gp41-Protein von HIV,
ausgewählt wird.

8. Verfahren nach Anspruch 1, wobei das erste Peptid durch Zugeben von wenigstens einer Aminosäure mit einer anionischen Seitenkette modifiziert wird.

9. Verfahren nach Anspruch 8, wobei die wenigstens eine Aminosäure mit einer anionischen Seitenkette aus der Gruppe, bestehend aus Glutamat und Aspartat, ausgewählt ist.

10. Verfahren nach Anspruch 1, wobei das erste Peptid an einer Seitenkette eines Aminosäurerestes modifiziert wird.

11. Verfahren nach Anspruch 10, wobei die Seitenkette in einer Weise modifiziert wird, die ausgewählt wird aus der Gruppe, bestehend aus:
a) Phosphorylierung von Serin, Threonin, Tyrosin, Aspartat oder Histidin;
b) Sulfatierung von Serin, Threonin, Tyrosin, Aspartat oder Histidin;
c) Carboxylierung von Glutamat;
d) Acylierung von Lysin und
e) Oxidation von Cystein.

12. Verfahren nach Anspruch 1, wobei das erste Peptid an wenigstens einem seiner N-terminalen oder C-terminalen Reste modifiziert wird.

13. Verfahren nach Anspruch 12, wobei das erste Peptid ein amidiertes Peptid ist und durch Carboxylierung des C-terminalen Restes modifiziert wird.

14. Verfahren nach Anspruch 12, wobei das erste Peptid durch Acylierung der N-terminalen Aminogruppe modifiziert wird.

15. Verfahren nach Anspruch 14, wobei die Acylierung Acetylierung oder Succinylierung ist.

16. Verfahren nach Anspruch 1, wobei die Vielzahl des zweiten Peptids unter Verwendung eines Mittels, ausgewählt aus der Gruppe, bestehend aus Sulfosuccinimidyl-4-(N-maleimidomethyl)cyclohexan-1-carboxylat (sSMCC), N-Maleimidobenzoyl-N-hydroxysuccinimidester (MBS), Glutaraldehyd, 1-Ethyl-3-(3-dimethylaminopropyl)carbodimid (EDCl), Bisdiazobenzidin (BDB) oder N-Acetylhomocysteinthiolacton (NAHT), N-Maleimidobenzoyl-N-hydroxysuccinimidester (MBS), Glutaraldehyd, 1-Ethyl-3-(3-dimethylaminopropyl)carbodiimid (EDCl), Bisdiazobenzidin (BDB) und N-Acetylhomocysteinthiolacton (NAHT), an den Träger konjugiert wird.

17. Verfahren nach Anspruch 16, wobei das Mittel sSMCC ist.

18. Verfahren nach Anspruch 1, wobei das erste Peptid ein Molekulargewicht im Bereich von 500 Da bis 30000 Da besitzt.

19. Verfahren nach Anspruch 1, wobei das erste Peptid ein Molekulargewicht im Bereich von 1400 Da bis 7900 Da besitzt.

20. Verfahren nach einem der Ansprüche 1-19, wobei die Peptidbeladung erhöht wird.

21. Verfahren nach Anspruch 1, wobei die Peptidbeladung auf mehr als 500 Mol Peptid/Mol Trägerprotein erhöht wird.

22. Verfahren nach Anspruch 1, wobei die Peptidbeladung auf mehr als 1000 Mol Peptid/Mol Trägerprotein erhöht wird.

23. Verfahren zur Bestimmung eines günstigen pl-Bereichs zur Konjugation eines Peptids an ein OMPC-Trägerprotein, umfassend:
a) Konjugieren einer Vielzahl des Peptids an den Träger, wobei jedes der Peptide so modifiziert wurde, dass es eine Vielzahl von verschiedenen pI gibt, und
b) Messen der Peptidbeladung für jedes der Peptide,
wobei der günstige pl-Bereich durch die pl der Peptide, die die günstigste Peptidbeladung besitzen, bestimmt wird.

24. Verfahren nach Anspruch 23, wobei der günstige pI-Bereich durch die pI jener Peptide, die eine Peptidbeladung von mehr als 500 Mol Peptid/Mol Trägerprotein besitzen, bestimmt wird.

25. Verfahren nach Anspruch 23, wobei der günstige pI-Bereich durch die pl jener Peptide, die eine Peptidbeladung von mehr als 1000 Mol Peptid/Mol Trägerprotein besitzen, bestimmt wird.

## Revendications

1. Procédé de production d'un conjugué porteur de peptides, comprenant:
a) modifier un premier peptide pour produire un deuxième peptide qui a un point isoélectrique (pI) plus bas que le premier peptide; et
b) conjuguer une pluralité du deuxième peptide avec une protéine porteuse de complexes protéique de membrane externe (OMPC) pour obtenir un conjugué porteur de peptides,
où au moins l'une d'entre la charge peptidique et la solubilité du conjugué consistant en la pluralité du deuxième peptide et à protéine porteuse d'OMPC, est augmentes par rapport à un conjugué d'une pluralité du premier peptide et de la protéine porteuse d'OMPC et ou le deuxième peptide a une séquence qui ne survient pas naturellement.

2. Le procédé de la revendication 1, dans lequel le deuxième peptide a un pI qui est inférieur à 6.

3. Le procédé de la revendication 1, dans lequel le deuxième peptide a un pI qui est entre 2 et 6,

4. Le procédé de la revendication 1, dans lequel le deuxième peptide a un pI qui est entre 3,5 et 5.

5. Le procédé de la revendication 1, dans lequel le premier peptide a une séquence d'acides aminés sélectionnés d'un pathogène.

6. Le procédé de la revendication 5, dans lequel le pathogène est sélectionné parmi le groupe consistant en *Haemophilus influenzae,* virus de l'hépatite A, B ou C, VIH, virus du papillome humain, rougeole, oreillons, rubéole, varicelle, rotavirus, *Streptococcus pneumoniae* et *Staphylococcus aureus*.

7. Le procédé de la revendication 1, dans lequel le premier peptide a une séquence d'acides aminés sélectionnée parmi le groupe consistant en:
a) la protéine HA du virus influenza A ou 13; et
b) à protéine gp41 du VIH.

8. Le procédé de la revendication 1 dans lequel le premier peptide est modifié en ajoutant au moins un acide aminé avec une chaîne latérale anionique.

9. Le procédé de à revendication 8, dans lequel le au moins un acide aminé avec une chaîne latérale anionique est sélectionné parmi le groupe consistant en glutamate et aspartate.

10. Le procédé de la revendication 1, dans lequel le premier peptide est modifié au niveau d'une chaîne latérale d'un résidu d'acide aminé.

11. Le procédé de la revendication 10, dans lequel la chaîne latérale est modifiée d'une manière sélectionnée parmi le groupe consistant en:
a) phosphorylation de sérine, thréonine, tyrosine, aspartate ou histidine;
b) sulfatation de sérine, thréonine, tyrosine, aspartate ou histidine;
c) carboxylation de glutamate;
d) acylation de lysine; et
e) oxydation de cystéine.

12. Le procédé de la revendication 1, dans lequel le premier peptide est modifié au niveau d'au moins l'un de ses résidus N-terminal ou C-terminal.

13. Le procède de la revendication 12, dans lequel le premier peptide est un peptide amidé et est modifié par la carboxylation du résidu C-terminal.

14. Le procédé de la revendication 12, dans lequel le premier peptide est modifié par l'acylation du groupe amino N-terminal.

15. Le procède de la revendication 14, dans lequel l'acylation est l'acétylation ou la succinylation.

16. Le procédé de la revendication 1, dans lequel la pluralité du deuxième peptide est conjuguée au porteur en utilisant un agent sélectionné parmi le groupe consistant en sulfosuccinidyl-4-(N-maléimidométhyl)cyclohexane-1-carboxylate (sSMCC), ester de N-maléimidobenzoyl-N-hydroxysuccinimide (MBS), glutaraldéhyde, 1-éthyl-3-(3-diméthylaminopropyl)carbodiimide (EDCI), bis-diazobenzidine (BDB) ou N-acétyl-homocystéine-thiolactone (NAHT), ester de N-maléimidobenzocyl-N-hydroxysuccinimide (MBS), glutaraldéhyde, 1-éthyl-3-(3-diméthylaminopropyl)carbodiimide (EDCI), bis-diaxobenxidine (BDB) et N-acétyl-homocystéine-thiolactone (NAHT)_{.}

17. Le procédé de la revendication 16, dans lequel l'agent est du sSMCC.

18. Le procédé de la revendication 1, dans lequel le premier peptide a un poids moléculaire dans la plage de 500 daltons à 30000 daltons.

19. Le procédé de à revendication 1, dans lequel le premier peptide a un poids moléculaire dans la plage de 1400 daltons à 7900 daltons.

20. Le procédé de l'une quelconque des revendications 1-19, dans lequel la charge peptidique est augmentée.

21. Le procédé de la revendication 1, dans lequel la charge peptidique est augmentée à plus de 500 moles de peptide/mole de protéine porteuse.

22. Le procédé de la revendication 1, dans lequel la charge peptidique est augmentée à plus de 1000 moles de peptide/mole de protéine porteuse.

23. Procédé de détermination d'une plage de pI favorable pour conjuguer un peptide avec une protéine porteuse d'OMPC, comprenant:
a) conjuguer une pluralité du peptide avec le porteur, chacun des peptides ayant été modifié d'une telle manière qu'il y a une pluralité de pI différents; et
b) mesurer la charge peptidique pour chacun des peptides,
où la plage de pI favorable est déterminée par les pI des peptides ayant la charge peptidique la plus favorable.

24. Le procédé de la revendication 23, dans lequel la plage de pI favorable est déterminée par les pI de ces peptides ayant une charge peptidiqie de plus de 500 moles de peptide/mole de protéine porteuse.

25. Le procédé de la revendication 23, dans lequel la plage de pI favorable est déterminée par les pI de ces peptides ayant une charge peptidique de plus de 1000 moles de peptide/mole de protéine porteuse.
